# EUROPEAN PATENT APPLICATION

(11) **EP 1 873 527 A1**
(43) Date of publication of application: **02.01.2008**
(21) Application number: 06013655.3
(22) Date of filing: 30.06.2006
(51) Int. Cl.: G01N 33/68, C12N 5/00, A01K 67/027, A61K 31/13

(54) **Method for identifying CRMP modulators**

(71) Applicant: SCHWARZ PHARMA AG, 40789 Monheim (DE)
(72) Inventor: Beyreuther, Bettina, Dr.rer.nat, 40219 Düsseldorf (DE); Stöhr, Thomas, Dr.rer.nat, 40789 Monheim (DE); Freitag, Joachim, Dr., 72622 Nuertingen (DE)
(74) Representative: Weiss, Wolfgang

(57) **Abstract**

The present invention refers to a method for identifying a modulator of CRMP suitable for the prevention, alleviation or/and treatment of CRMP-associated diseases.

SEQUENCE LISTING

<110>  Schwarz Pharma AG

<120>  Method for identifying CRMP modulators

<130>  34509P EP

<160>  12

<170>  PatentIn version 3.3

<210>  1
<211>  2061
<212>  DNA
<213>  Homo sapiens

<220>
<221>  CDS
<222>  (1)..(2061)
<223>  CRMP-1 splice variant 1

<400>  1
atg gcg gac cgg cgg cgc gcg tgg aac acc gag gac gac ctg ccc gtg      48
Met Ala Asp Arg Arg Arg Ala Trp Asn Thr Glu Asp Asp Leu Pro Val
1               5                   10                  15

tac ctg gcg cgg ccg ggc agc gcg gcg cag acc ccg cgc cag aag tac      96
Tyr Leu Ala Arg Pro Gly Ser Ala Ala Gln Thr Pro Arg Gln Lys Tyr
            20                  25                  30

ggc ggc atg ttc gcc gcg gtg gag ggc gcc tac gag aac aag acc atc      144
Gly Gly Met Phe Ala Ala Val Glu Gly Ala Tyr Glu Asn Lys Thr Ile
        35                  40                  45

gac ttc gac gcc tac agt gtg ggc cgc cgc ggc tcg gcg cgc acg ccg      192
Asp Phe Asp Ala Tyr Ser Val Gly Arg Arg Gly Ser Ala Arg Thr Pro
    50                  55                  60

cgc agc gct ggc cgg ccc gac gcg gtc ggg ctg cca ggg ccg gga ggc      240
Arg Ser Ala Gly Arg Pro Asp Ala Val Gly Leu Pro Gly Pro Gly Gly
65                  70                  75                  80

agc gag gac acg gcc agc gac gtg agc gag ccc tcg ggc tcc gcg gtc      288
Ser Glu Asp Thr Ala Ser Asp Val Ser Glu Pro Ser Gly Ser Ala Val
                85                  90                  95

agc tct ccc gga gag cgc gac gag cgg ccg ccg acg ctg cgc atc cgc      336
Ser Ser Pro Gly Glu Arg Asp Glu Arg Pro Pro Thr Leu Arg Ile Arg
            100                 105                 110

cgc ccc gcg ccc cgc gac ctg ccc ctc ggc cgg gac aat ggc cag agt      384
Arg Pro Ala Pro Arg Asp Leu Pro Leu Gly Arg Asp Asn Gly Gln Ser
        115                 120                 125

gac cga ctc ctc atc aaa ggt gga cgg atc atc aac gat gac caa tcc      432
Asp Arg Leu Leu Ile Lys Gly Gly Arg Ile Ile Asn Asp Asp Gln Ser

130                 135                 140

ctt tat gct gac gtc tac ctg gag gat gga ctt atc aaa caa ata gga        480
Leu Tyr Ala Asp Val Tyr Leu Glu Asp Gly Leu Ile Lys Gln Ile Gly
145                 150                 155                 160

gag aac tta atc gtt cct ggt gga gtg aag acc att gaa gcc aac ggg        528
Glu Asn Leu Ile Val Pro Gly Gly Val Lys Thr Ile Glu Ala Asn Gly
                165                 170                 175

cgg atg gtt att ccc gga ggt att gat gtc aac acg tac ctg cag aag        576
Arg Met Val Ile Pro Gly Gly Ile Asp Val Asn Thr Tyr Leu Gln Lys
            180                 185                 190

ccc tcc cag ggg atg act gcg gct gat gac ttc ttc caa ggg acc agg        624
Pro Ser Gln Gly Met Thr Ala Ala Asp Asp Phe Phe Gln Gly Thr Arg
        195                 200                 205

gcg gca ctg gtg ggc ggg acc acg atg atc att gac cat gtt gtt cct        672
Ala Ala Leu Val Gly Gly Thr Thr Met Ile Ile Asp His Val Val Pro
    210                 215                 220

gaa cct ggg tcc agc cta ctg acc tct ttc gag aag tgg cac gaa gca        720
Glu Pro Gly Ser Ser Leu Leu Thr Ser Phe Glu Lys Trp His Glu Ala
225                 230                 235                 240

gct gac acc aaa tcc tgc tgt gat tac tcc ctc cac gtg gac atc aca        768
Ala Asp Thr Lys Ser Cys Cys Asp Tyr Ser Leu His Val Asp Ile Thr
                245                 250                 255

agc tgg tac gat ggc gtt cgg gag gag ctg gag gtg ctg gtg cag gac        816
Ser Trp Tyr Asp Gly Val Arg Glu Glu Leu Glu Val Leu Val Gln Asp
            260                 265                 270

aaa ggc gtc aat tcc ttc caa gtc tac atg gcc tat aag gat gtc tac        864
Lys Gly Val Asn Ser Phe Gln Val Tyr Met Ala Tyr Lys Asp Val Tyr
        275                 280                 285

caa atg tcc gac agc cag ctc tat gaa gcc ttt acc ttc ctt aag ggc        912
Gln Met Ser Asp Ser Gln Leu Tyr Glu Ala Phe Thr Phe Leu Lys Gly
    290                 295                 300

ctg gga gct gtg atc ttg gtc cat gca gaa aat gga gat ttg ata gct        960
Leu Gly Ala Val Ile Leu Val His Ala Glu Asn Gly Asp Leu Ile Ala
305                 310                 315                 320

cag gaa caa aag cgg atc ctg gag atg ggc atc acg ggt ccc gag ggc       1008
Gln Glu Gln Lys Arg Ile Leu Glu Met Gly Ile Thr Gly Pro Glu Gly
                325                 330                 335

cat gcc ctg agc aga cct gaa gag ctg gag gcc gag gcg gtg ttc cgg       1056
His Ala Leu Ser Arg Pro Glu Glu Leu Glu Ala Glu Ala Val Phe Arg
            340                 345                 350

gcc atc acc att gcg ggc cgg atc aac tgc cct gtg tac atc acc aag       1104
Ala Ile Thr Ile Ala Gly Arg Ile Asn Cys Pro Val Tyr Ile Thr Lys
        355                 360                 365

gtc atg agc aag agt gca gcc gac atc atc gct ctg gcc agg aag aaa     1152
Val Met Ser Lys Ser Ala Ala Asp Ile Ile Ala Leu Ala Arg Lys Lys
    370                 375                 380

ggg ccc cta gtt ttt gga gag ccc att gcc gcc agc ctg ggg acc gat     1200
Gly Pro Leu Val Phe Gly Glu Pro Ile Ala Ala Ser Leu Gly Thr Asp
385                 390                 395                 400

ggc acc cat tac tgg agc aag aac tgg gcc aag gct gcg gcg ttc gtg     1248
Gly Thr His Tyr Trp Ser Lys Asn Trp Ala Lys Ala Ala Ala Phe Val
                405                 410                 415

act tcc cct ccc ctg agc ccg gac cct acc acg ccc gac tac ttg acc     1296
Thr Ser Pro Pro Leu Ser Pro Asp Pro Thr Thr Pro Asp Tyr Leu Thr
            420                 425                 430

tcc cta ctg gcc tgt ggg gac ttg cag gtc aca ggc agc ggc cac tgt     1344
Ser Leu Leu Ala Cys Gly Asp Leu Gln Val Thr Gly Ser Gly His Cys
        435                 440                 445

ccc tac agc act gcc cag aag gcg gtg ggc aag gac aac ttt acc ctg     1392
Pro Tyr Ser Thr Ala Gln Lys Ala Val Gly Lys Asp Asn Phe Thr Leu
    450                 455                 460

atc ccc gag ggt gtc aac ggg ata gag gag cgg atg acg gtc gtc tgg     1440
Ile Pro Glu Gly Val Asn Gly Ile Glu Glu Arg Met Thr Val Val Trp
465                 470                 475                 480

gac aag gcg gtg gct act ggc aaa atg gat gag aac cag ttt gtc gct     1488
Asp Lys Ala Val Ala Thr Gly Lys Met Asp Glu Asn Gln Phe Val Ala
                485                 490                 495

gtc acc agc acc aat gca gcc aag atc ttt aac ctg tac cca agg aaa     1536
Val Thr Ser Thr Asn Ala Ala Lys Ile Phe Asn Leu Tyr Pro Arg Lys
            500                 505                 510

ggg cgg att gcc gtg ggc tcg gat gcc gac gtg gtc atc tgg gac ccc     1584
Gly Arg Ile Ala Val Gly Ser Asp Ala Asp Val Val Ile Trp Asp Pro
        515                 520                 525

gac aag ttg aag acc ata aca gcc aaa agt cac aag tcg gcg gtg gag     1632
Asp Lys Leu Lys Thr Ile Thr Ala Lys Ser His Lys Ser Ala Val Glu
    530                 535                 540

tac aac atc ttc gag ggt atg gag tgc cac ggc tcc cca cta gtg gtc     1680
Tyr Asn Ile Phe Glu Gly Met Glu Cys His Gly Ser Pro Leu Val Val
545                 550                 555                 560

atc agc cag ggc aag atc gtc ttt gaa gac gga aac atc aac gtc aac     1728
Ile Ser Gln Gly Lys Ile Val Phe Glu Asp Gly Asn Ile Asn Val Asn
                565                 570                 575

aag ggc atg ggc cgc ttc att ccg cgg aag gcg ttc ccg gag cac ctg     1776
Lys Gly Met Gly Arg Phe Ile Pro Arg Lys Ala Phe Pro Glu His Leu
            580                 585                 590

tac cag cgc gtc aaa atc agg aat aag gtt ttt gga ttg caa ggg gtt     1824
Tyr Gln Arg Val Lys Ile Arg Asn Lys Val Phe Gly Leu Gln Gly Val

595                 600                 605

tcc agg ggc atg tat gac ggt cct gtg tac gag gta cca gct aca ccc      1872
Ser Arg Gly Met Tyr Asp Gly Pro Val Tyr Glu Val Pro Ala Thr Pro
    610                 615                 620

aaa tat gca act ccc gct cct tca gcc aaa tct tcg cct tct aaa cac      1920
Lys Tyr Ala Thr Pro Ala Pro Ser Ala Lys Ser Ser Pro Ser Lys His
625                 630                 635                 640

cag ccc cca ccc atc aga aac ctc cac cag tcc aac ttc agc tta tca      1968
Gln Pro Pro Pro Ile Arg Asn Leu His Gln Ser Asn Phe Ser Leu Ser
                645                 650                 655

ggt gcc cag ata gat gac aac aat ccc agg cgc acc ggc cac cgc atc      2016
Gly Ala Gln Ile Asp Asp Asn Asn Pro Arg Arg Thr Gly His Arg Ile
            660                 665                 670

gtg gcg ccc cct ggt ggc cgc tcc aac atc acc agc ctc ggt tga          2061
Val Ala Pro Pro Gly Gly Arg Ser Asn Ile Thr Ser Leu Gly
        675                 680                 685


<210>  2
<211>  686
<212>  PRT
<213>  Homo sapiens

<400>  2

Met Ala Asp Arg Arg Arg Ala Trp Asn Thr Glu Asp Asp Leu Pro Val
1               5                   10                  15


Tyr Leu Ala Arg Pro Gly Ser Ala Ala Gln Thr Pro Arg Gln Lys Tyr
            20                  25                  30


Gly Gly Met Phe Ala Ala Val Glu Gly Ala Tyr Glu Asn Lys Thr Ile
        35                  40                  45


Asp Phe Asp Ala Tyr Ser Val Gly Arg Arg Gly Ser Ala Arg Thr Pro
    50                  55                  60


Arg Ser Ala Gly Arg Pro Asp Ala Val Gly Leu Pro Gly Pro Gly Gly
65                  70                  75                  80


Ser Glu Asp Thr Ala Ser Asp Val Ser Glu Pro Ser Gly Ser Ala Val
                85                  90                  95


Ser Ser Pro Gly Glu Arg Asp Glu Arg Pro Pro Thr Leu Arg Ile Arg
            100                 105                 110

Arg Pro Ala Pro Arg Asp Leu Pro Leu Gly Arg Asp Asn Gly Gln Ser
        115                 120                 125

Asp Arg Leu Leu Ile Lys Gly Gly Arg Ile Ile Asn Asp Asp Gln Ser
    130                 135                 140

Leu Tyr Ala Asp Val Tyr Leu Glu Asp Gly Leu Ile Lys Gln Ile Gly
145                 150                 155                 160

Glu Asn Leu Ile Val Pro Gly Gly Val Lys Thr Ile Glu Ala Asn Gly
                165                 170                 175

Arg Met Val Ile Pro Gly Gly Ile Asp Val Asn Thr Tyr Leu Gln Lys
            180                 185                 190

Pro Ser Gln Gly Met Thr Ala Ala Asp Asp Phe Phe Gln Gly Thr Arg
        195                 200                 205

Ala Ala Leu Val Gly Gly Thr Thr Met Ile Ile Asp His Val Val Pro
    210                 215                 220

Glu Pro Gly Ser Ser Leu Leu Thr Ser Phe Glu Lys Trp His Glu Ala
225                 230                 235                 240

Ala Asp Thr Lys Ser Cys Cys Asp Tyr Ser Leu His Val Asp Ile Thr
                245                 250                 255

Ser Trp Tyr Asp Gly Val Arg Glu Glu Leu Glu Val Leu Val Gln Asp
            260                 265                 270

Lys Gly Val Asn Ser Phe Gln Val Tyr Met Ala Tyr Lys Asp Val Tyr
        275                 280                 285

Gln Met Ser Asp Ser Gln Leu Tyr Glu Ala Phe Thr Phe Leu Lys Gly
    290                 295                 300

Leu Gly Ala Val Ile Leu Val His Ala Glu Asn Gly Asp Leu Ile Ala
305                 310                 315                 320

Gln Glu Gln Lys Arg Ile Leu Glu Met Gly Ile Thr Gly Pro Glu Gly
                325                 330                 335

His Ala Leu Ser Arg Pro Glu Glu Leu Glu Ala Glu Ala Val Phe Arg
            340                 345                 350

Ala Ile Thr Ile Ala Gly Arg Ile Asn Cys Pro Val Tyr Ile Thr Lys
        355                 360                 365

Val Met Ser Lys Ser Ala Ala Asp Ile Ile Ala Leu Ala Arg Lys Lys
    370                 375                 380

Gly Pro Leu Val Phe Gly Glu Pro Ile Ala Ala Ser Leu Gly Thr Asp
385                 390                 395                 400

Gly Thr His Tyr Trp Ser Lys Asn Trp Ala Lys Ala Ala Ala Phe Val
                405                 410                 415

Thr Ser Pro Pro Leu Ser Pro Asp Pro Thr Thr Pro Asp Tyr Leu Thr
            420                 425                 430

Ser Leu Leu Ala Cys Gly Asp Leu Gln Val Thr Gly Ser Gly His Cys
        435                 440                 445

Pro Tyr Ser Thr Ala Gln Lys Ala Val Gly Lys Asp Asn Phe Thr Leu
    450                 455                 460

Ile Pro Glu Gly Val Asn Gly Ile Glu Glu Arg Met Thr Val Val Trp
465                 470                 475                 480

Asp Lys Ala Val Ala Thr Gly Lys Met Asp Glu Asn Gln Phe Val Ala
                485                 490                 495

Val Thr Ser Thr Asn Ala Ala Lys Ile Phe Asn Leu Tyr Pro Arg Lys
            500                 505                 510

Gly Arg Ile Ala Val Gly Ser Asp Ala Asp Val Val Ile Trp Asp Pro
        515                 520                 525

Asp Lys Leu Lys Thr Ile Thr Ala Lys Ser His Lys Ser Ala Val Glu
    530                 535                 540

Tyr Asn Ile Phe Glu Gly Met Glu Cys His Gly Ser Pro Leu Val Val
545                 550                 555                 560

Ile Ser Gln Gly Lys Ile Val Phe Glu Asp Gly Asn Ile Asn Val Asn
                565                 570                 575

Lys Gly Met Gly Arg Phe Ile Pro Arg Lys Ala Phe Pro Glu His Leu
            580                 585                 590

Tyr Gln Arg Val Lys Ile Arg Asn Lys Val Phe Gly Leu Gln Gly Val
        595                 600                 605

Ser Arg Gly Met Tyr Asp Gly Pro Val Tyr Glu Val Pro Ala Thr Pro
    610                 615                 620

Lys Tyr Ala Thr Pro Ala Pro Ser Ala Lys Ser Ser Pro Ser Lys His
625                 630                 635                 640

Gln Pro Pro Pro Ile Arg Asn Leu His Gln Ser Asn Phe Ser Leu Ser
            645                 650                 655

Gly Ala Gln Ile Asp Asp Asn Asn Pro Arg Arg Thr Gly His Arg Ile
            660                 665                 670

Val Ala Pro Pro Gly Gly Arg Ser Asn Ile Thr Ser Leu Gly
        675                 680                 685

<210>  3
<211>  1719
<212>  DNA
<213>  Homo sapiens

<220>
<221>  CDS
<222>  (1)..(1719)
<223>  CRMP-1 splice variant 2

<400>  3
atg tcg tac cag ggc aag aag agc atc ccg cac atc acg agt gac cga      48
Met Ser Tyr Gln Gly Lys Lys Ser Ile Pro His Ile Thr Ser Asp Arg
1               5                   10                  15

ctc ctc atc aaa ggt gga cgg atc atc aac gat gac caa tcc ctt tat      96
Leu Leu Ile Lys Gly Gly Arg Ile Ile Asn Asp Asp Gln Ser Leu Tyr
            20                  25                  30

gct gac gtc tac ctg gag gat gga ctt atc aaa caa ata gga gag aac     144
Ala Asp Val Tyr Leu Glu Asp Gly Leu Ile Lys Gln Ile Gly Glu Asn
        35                  40                  45

tta atc gtt cct ggt gga gtg aag acc att gaa gcc aac ggg cgg atg     192
Leu Ile Val Pro Gly Gly Val Lys Thr Ile Glu Ala Asn Gly Arg Met
    50                  55                  60

gtt att ccc gga ggt att gat gtc aac acg tac ctg cag aag ccc tcc     240
Val Ile Pro Gly Gly Ile Asp Val Asn Thr Tyr Leu Gln Lys Pro Ser

65                  70                  75                  80

cag ggg atg act gcg gct gat gac ttc ttc caa ggg acc agg gcg gca          288
Gln Gly Met Thr Ala Ala Asp Asp Phe Phe Gln Gly Thr Arg Ala Ala
                85                  90                  95

ctg gtg ggc ggg acc acg atg atc att gac cat gtt gtt cct gaa cct          336
Leu Val Gly Gly Thr Thr Met Ile Ile Asp His Val Val Pro Glu Pro
            100                 105                 110

ggg tcc agc cta ctg acc tct ttc gag aag tgg cac gaa gca gct gac          384
Gly Ser Ser Leu Leu Thr Ser Phe Glu Lys Trp His Glu Ala Ala Asp
        115                 120                 125

acc aaa tcc tgc tgt gat tac tcc ctc cac gtg gac atc aca agc tgg          432
Thr Lys Ser Cys Cys Asp Tyr Ser Leu His Val Asp Ile Thr Ser Trp
    130                 135                 140

tac gat ggc gtt cgg gag gag ctg gag gtg ctg gtg cag gac aaa ggc          480
Tyr Asp Gly Val Arg Glu Glu Leu Glu Val Leu Val Gln Asp Lys Gly
145                 150                 155                 160

gtc aat tcc ttc caa gtc tac atg gcc tat aag gat gtc tac caa atg          528
Val Asn Ser Phe Gln Val Tyr Met Ala Tyr Lys Asp Val Tyr Gln Met
                165                 170                 175

tcc gac agc cag ctc tat gaa gcc ttt acc ttc ctt aag ggc ctg gga          576
Ser Asp Ser Gln Leu Tyr Glu Ala Phe Thr Phe Leu Lys Gly Leu Gly
            180                 185                 190

gct gtg atc ttg gtc cat gca gaa aat gga gat ttg ata gct cag gaa          624
Ala Val Ile Leu Val His Ala Glu Asn Gly Asp Leu Ile Ala Gln Glu
        195                 200                 205

caa aag cgg atc ctg gag atg ggc atc acg ggt ccc gag ggc cat gcc          672
Gln Lys Arg Ile Leu Glu Met Gly Ile Thr Gly Pro Glu Gly His Ala
    210                 215                 220

ctg agc aga cct gaa gag ctg gag gcc gag gcg gtg ttc cgg gcc atc          720
Leu Ser Arg Pro Glu Glu Leu Glu Ala Glu Ala Val Phe Arg Ala Ile
225                 230                 235                 240

acc att gcg ggc cgg atc aac tgc cct gtg tac atc acc aag gtc atg          768
Thr Ile Ala Gly Arg Ile Asn Cys Pro Val Tyr Ile Thr Lys Val Met
                245                 250                 255

agc aag agt gca gcc gac atc atc gct ctg gcc agg aag aaa ggg ccc          816
Ser Lys Ser Ala Ala Asp Ile Ile Ala Leu Ala Arg Lys Lys Gly Pro
            260                 265                 270

cta gtt ttt gga gag ccc att gcc gcc agc ctg ggg acc gat ggc acc          864
Leu Val Phe Gly Glu Pro Ile Ala Ala Ser Leu Gly Thr Asp Gly Thr
        275                 280                 285

cat tac tgg agc aag aac tgg gcc aag gct gcg gcg ttc gtg act tcc          912
His Tyr Trp Ser Lys Asn Trp Ala Lys Ala Ala Ala Phe Val Thr Ser
    290                 295                 300

cct ccc ctg agc ccg gac cct acc acg ccc gac tac ttg acc tcc cta 960
Pro Pro Leu Ser Pro Asp Pro Thr Thr Pro Asp Tyr Leu Thr Ser Leu
305                 310                 315                 320

ctg gcc tgt ggg gac ttg cag gtc aca ggc agc ggc cac tgt ccc tac 1008
Leu Ala Cys Gly Asp Leu Gln Val Thr Gly Ser Gly His Cys Pro Tyr
                325                 330                 335

agc act gcc cag aag gcg gtg ggc aag gac aac ttt acc ctg atc ccc 1056
Ser Thr Ala Gln Lys Ala Val Gly Lys Asp Asn Phe Thr Leu Ile Pro
            340                 345                 350

gag ggt gtc aac ggg ata gag gag cgg atg acg gtc gtc tgg gac aag 1104
Glu Gly Val Asn Gly Ile Glu Glu Arg Met Thr Val Val Trp Asp Lys
        355                 360                 365

gcg gtg gct act ggc aaa atg gat gag aac cag ttt gtc gct gtc acc 1152
Ala Val Ala Thr Gly Lys Met Asp Glu Asn Gln Phe Val Ala Val Thr
    370                 375                 380

agc acc aat gca gcc aag atc ttt aac ctg tac cca agg aaa ggg cgg 1200
Ser Thr Asn Ala Ala Lys Ile Phe Asn Leu Tyr Pro Arg Lys Gly Arg
385                 390                 395                 400

att gcc gtg ggc tcg gat gcc gac gtg gtc atc tgg gac ccc gac aag 1248
Ile Ala Val Gly Ser Asp Ala Asp Val Val Ile Trp Asp Pro Asp Lys
                405                 410                 415

ttg aag acc ata aca gcc aaa agt cac aag tcg gcg gtg gag tac aac 1296
Leu Lys Thr Ile Thr Ala Lys Ser His Lys Ser Ala Val Glu Tyr Asn
            420                 425                 430

atc ttc gag ggt atg gag tgc cac ggc tcc cca cta gtg gtc atc agc 1344
Ile Phe Glu Gly Met Glu Cys His Gly Ser Pro Leu Val Val Ile Ser
        435                 440                 445

cag ggc aag atc gtc ttt gaa gac gga aac atc aac gtc aac aag ggc 1392
Gln Gly Lys Ile Val Phe Glu Asp Gly Asn Ile Asn Val Asn Lys Gly
    450                 455                 460

atg ggc cgc ttc att ccg cgg aag gcg ttc ccg gag cac ctg tac cag 1440
Met Gly Arg Phe Ile Pro Arg Lys Ala Phe Pro Glu His Leu Tyr Gln
465                 470                 475                 480

cgc gtc aaa atc agg aat aag gtt ttt gga ttg caa ggg gtt tcc agg 1488
Arg Val Lys Ile Arg Asn Lys Val Phe Gly Leu Gln Gly Val Ser Arg
                485                 490                 495

ggc atg tat gac ggt cct gtg tac gag gta cca gct aca ccc aaa tat 1536
Gly Met Tyr Asp Gly Pro Val Tyr Glu Val Pro Ala Thr Pro Lys Tyr
            500                 505                 510

gca act ccc gct cct tca gcc aaa tct tcg cct tct aaa cac cag ccc 1584
Ala Thr Pro Ala Pro Ser Ala Lys Ser Ser Pro Ser Lys His Gln Pro
        515                 520                 525

cca ccc atc aga aac ctc cac cag tcc aac ttc agc tta tca ggt gcc 1632
Pro Pro Ile Arg Asn Leu His Gln Ser Asn Phe Ser Leu Ser Gly Ala

530                    535                    540

cag ata gat gac aac aat ccc agg cgc acc ggc cac cgc atc gtg gcg      1680
Gln Ile Asp Asp Asn Asn Pro Arg Arg Thr Gly His Arg Ile Val Ala
545                 550                 555                 560

ccc cct ggt ggc cgc tcc aac atc acc agc ctc ggt tga                  1719
Pro Pro Gly Gly Arg Ser Asn Ile Thr Ser Leu Gly
                565                 570

<210>  4
<211>  572
<212>  PRT
<213>  Homo sapiens

<400>  4

Met Ser Tyr Gln Gly Lys Lys Ser Ile Pro His Ile Thr Ser Asp Arg
1               5                   10                  15

Leu Leu Ile Lys Gly Gly Arg Ile Ile Asn Asp Asp Gln Ser Leu Tyr
            20                  25                  30

Ala Asp Val Tyr Leu Glu Asp Gly Leu Ile Lys Gln Ile Gly Glu Asn
        35                  40                  45

Leu Ile Val Pro Gly Gly Val Lys Thr Ile Glu Ala Asn Gly Arg Met
    50                  55                  60

Val Ile Pro Gly Gly Ile Asp Val Asn Thr Tyr Leu Gln Lys Pro Ser
65                  70                  75                  80

Gln Gly Met Thr Ala Ala Asp Asp Phe Phe Gln Gly Thr Arg Ala Ala
                85                  90                  95

Leu Val Gly Gly Thr Thr Met Ile Ile Asp His Val Val Pro Glu Pro
            100                 105                 110

Gly Ser Ser Leu Leu Thr Ser Phe Glu Lys Trp His Glu Ala Ala Asp
        115                 120                 125

Thr Lys Ser Cys Cys Asp Tyr Ser Leu His Val Asp Ile Thr Ser Trp
    130                 135                 140

Tyr Asp Gly Val Arg Glu Glu Leu Glu Val Leu Val Gln Asp Lys Gly
145                 150                 155                 160

Val Asn Ser Phe Gln Val Tyr Met Ala Tyr Lys Asp Val Tyr Gln Met
                165                 170                 175

Ser Asp Ser Gln Leu Tyr Glu Ala Phe Thr Phe Leu Lys Gly Leu Gly
            180                 185                 190

Ala Val Ile Leu Val His Ala Glu Asn Gly Asp Leu Ile Ala Gln Glu
        195                 200                 205

Gln Lys Arg Ile Leu Glu Met Gly Ile Thr Gly Pro Glu Gly His Ala
    210                 215                 220

Leu Ser Arg Pro Glu Glu Leu Glu Ala Glu Ala Val Phe Arg Ala Ile
225                 230                 235                 240

Thr Ile Ala Gly Arg Ile Asn Cys Pro Val Tyr Ile Thr Lys Val Met
                245                 250                 255

Ser Lys Ser Ala Ala Asp Ile Ile Ala Leu Ala Arg Lys Lys Gly Pro
            260                 265                 270

Leu Val Phe Gly Glu Pro Ile Ala Ala Ser Leu Gly Thr Asp Gly Thr
        275                 280                 285

His Tyr Trp Ser Lys Asn Trp Ala Lys Ala Ala Ala Phe Val Thr Ser
    290                 295                 300

Pro Pro Leu Ser Pro Asp Pro Thr Thr Pro Asp Tyr Leu Thr Ser Leu
305                 310                 315                 320

Leu Ala Cys Gly Asp Leu Gln Val Thr Gly Ser Gly His Cys Pro Tyr
                325                 330                 335

Ser Thr Ala Gln Lys Ala Val Gly Lys Asp Asn Phe Thr Leu Ile Pro
            340                 345                 350

Glu Gly Val Asn Gly Ile Glu Glu Arg Met Thr Val Val Trp Asp Lys
        355                 360                 365

Ala Val Ala Thr Gly Lys Met Asp Glu Asn Gln Phe Val Ala Val Thr
    370                 375                 380

Ser Thr Asn Ala Ala Lys Ile Phe Asn Leu Tyr Pro Arg Lys Gly Arg
385                 390                 395                 400

Ile Ala Val Gly Ser Asp Ala Asp Val Val Ile Trp Asp Pro Asp Lys
                405                 410                 415

Leu Lys Thr Ile Thr Ala Lys Ser His Lys Ser Ala Val Glu Tyr Asn
            420                 425                 430

Ile Phe Glu Gly Met Glu Cys His Gly Ser Pro Leu Val Val Ile Ser
        435                 440                 445

Gln Gly Lys Ile Val Phe Glu Asp Gly Asn Ile Asn Val Asn Lys Gly
    450                 455                 460

Met Gly Arg Phe Ile Pro Arg Lys Ala Phe Pro Glu His Leu Tyr Gln
465                 470                 475                 480

Arg Val Lys Ile Arg Asn Lys Val Phe Gly Leu Gln Gly Val Ser Arg
                485                 490                 495

Gly Met Tyr Asp Gly Pro Val Tyr Glu Val Pro Ala Thr Pro Lys Tyr
            500                 505                 510

Ala Thr Pro Ala Pro Ser Ala Lys Ser Ser Pro Ser Lys His Gln Pro
        515                 520                 525

Pro Pro Ile Arg Asn Leu His Gln Ser Asn Phe Ser Leu Ser Gly Ala
    530                 535                 540

Gln Ile Asp Asp Asn Asn Pro Arg Arg Thr Gly His Arg Ile Val Ala
545                 550                 555                 560

Pro Pro Gly Gly Arg Ser Asn Ile Thr Ser Leu Gly
                565                 570

<210>  5
<211>  1719
<212>  DNA
<213>  Homo sapiens

<220>
<221>  CDS
<222>  (1)..(1719)
<223>  CRMP-2

<220>

<221>  misc_feature
<222>  (426)..(426)
<223>  C at position 426 can be C or T, no amino acid change

<400>  5
atg tct tat cag ggg aag aaa aat att cca cgc atc acg agc gat cgt      48
ctt ctg atc aaa gga ggt aaa att gtt aat gat gac cag tcg ttc tat      96
gca gac ata tac atg gaa gat ggg ttg atc aag caa ata gga gaa aat      144
ctg att gtg cca gga gga gtg aag acc atc gag gcc cac tcc cgg atg      192
gtg atc ccc gga gga att gac gtc cac act cgt ttc cag atg cct gat      240
cag gga atg acg tct gct gat gat ttc ttc caa gga acc aag gcg gcc      288
ctg gct ggg gga acc act atg atc att gac cac gtt gtt cct gag cct      336
ggg aca agc ctg ctc gct gcc ttt gac cag tgg agg gaa tgg gcc gac      384
agc aag tcc tgc tgt gac tac tct ctg cat gtg gac atc agc gag tgg      432
cat aag ggc atc cag gag gag atg gaa gcg ctt gtg aag gat cac ggg      480
gta aat tcc ttc ctc gtg tac atg gct ttc aaa gat cgc ttc cag cta      528
acg gat tgc cag att tat gaa gta ctg agt gtg atc cgg gat att ggc      576
gcc ata gcc caa gtc cac gca gaa aat ggc gac atc att gca gag gag      624
cag cag agg atc ctg gat ctg ggc atc acg ggc ccc gag gga cat gtg      672
ctg agc cga cct gag gag gtc gag gcc gaa gcc gtg aat cgt gcc atc 720
acc atc gcc aac cag acc aac tgc ccg ctg tat atc acc aag gtg atg 768
agc aaa agc tct gct gag gtc atc gcc cag gca cgg aag aag gga act 816
gtg gtg tat ggc gag ccc atc act gcc agc ttg gga acg gac ggc tcc 864
cat tac tgg agc aag aac tgg gcc aag gct gct gcc ttt gtc acc tcc 912
cca ccc ttg agc cct gat cca acc act cca gac ttt ctc aac tcc ttg 960
ctg tcc tgt gga gac ctc cag gtc acg ggc agt gcc cat tgc acg ttt 1008
aac act gcc cag aag gct gta gga aag gac aac ttc acc ctg att ccg 1056
gag ggc acc aat ggc act gag gag cgg atg tcc gtc atc tgg gac aag 1104
gct gtg gtc act ggg aag atg gat gag aac cag ttt gtg gct gtg acc 1152
agc acc aat gca gcc aaa gtc ttc aac ctt tac ccc cgg aaa ggc cgc 1200
att gct gtg gga tcc gat gcc gac ctg gtc atc tgg gac ccc gac agc 1248
gtt aaa acc atc tct gcc aag aca cac aac agc tct ctc gag tac aac 1296
atc ttt gaa ggc atg gag tgc cgc ggc tcc cca ctg gtg gtc atc agc 1344
cag ggg aag att gtc ctg gag gac ggc acc ctg cat gtc acc gaa ggc     1392
tct gga cgc tac att ccc cgg aag ccc ttc cct gat ttt gtt tac aag     1440
cgt atc aag gca agg agc agg ctg gct gag ctg aga ggg gtt cct cgt     1488
ggc ctg tat gac gga cct gtg tgt gaa gtg tct gtg acg ccc aag aca     1536
gtc act cca gcc tcc tcg gcc aag acg tct cct gcc aag cag cag gcc     1584
cca cct gtc cgg aac ctg cac cag tct gga ttc agt ttg tct ggt gct     1632
cag att gat gac aac att ccc cgc cgc acc acc cag cgt atc gtg gcg     1680
ccc ccc ggt ggc cgt gcc aac atc acc agc ctg ggc tag                 1719


<210>  6
<211>  572
<212>  PRT
<213>  Homo sapiens

<400>  6

<210>  7
<211>  1719
<212>  DNA
<213>  Homo sapiens

<220>
<221>  CDS
<222>  (1)..(1719)
<223>  CRMP-3

<400>  7
atg tcc ttc cag ggc aag aaa agc atc ccc cgg atc acg agt gac cgc      48
Met Ser Phe Gln Gly Lys Lys Ser Ile Pro Arg Ile Thr Ser Asp Arg
1               5                   10                  15

ctt ctg atc aga ggt ggg agg atc gtg aat gac gac cag tcc ttt tac      96
Leu Leu Ile Arg Gly Gly Arg Ile Val Asn Asp Asp Gln Ser Phe Tyr
            20                  25                  30

gct gat gtg cac gtg gaa gat ggc ttg ata aaa caa atc gga gaa aac     144
Ala Asp Val His Val Glu Asp Gly Leu Ile Lys Gln Ile Gly Glu Asn
        35                  40                  45

ctc atc gtc cct ggg ggc atc aag acc att gac gcc cac ggc ctg atg     192
Leu Ile Val Pro Gly Gly Ile Lys Thr Ile Asp Ala His Gly Leu Met
    50                  55                  60

gtc ctt cct ggt ggc gtt gac gtc cac aca agg ctg cag atg cct gtc     240
Val Leu Pro Gly Gly Val Asp Val His Thr Arg Leu Gln Met Pro Val
65                  70                  75                  80

ctg ggc atg aca ccg gct gac gac ttc tgt cag ggc acc aag gca gcg     288
Leu Gly Met Thr Pro Ala Asp Asp Phe Cys Gln Gly Thr Lys Ala Ala
                85                  90                  95

cta gca gga gga acc acc atg atc ttg gac cac gtc ttc ccc gac acg     336
Leu Ala Gly Gly Thr Thr Met Ile Leu Asp His Val Phe Pro Asp Thr
            100                 105                 110

ggt gtg agc ctg ctg gcg gcc tac gag cgg tgg cgg gag cgg gcg gac     384
Gly Val Ser Leu Leu Ala Ala Tyr Glu Arg Trp Arg Glu Arg Ala Asp
        115                 120                 125

agc gcg gcc tgc tgc gac tac tcc ctg cac gtg gac atc acc cga tgg     432
Ser Ala Ala Cys Cys Asp Tyr Ser Leu His Val Asp Ile Thr Arg Trp
    130                 135                 140

cat gag agc atc aag gag gag ctg gag gcc ctg gtc aag gag aag ggt 480
His Glu Ser Ile Lys Glu Glu Leu Glu Ala Leu Val Lys Glu Lys Gly
145                 150                 155                 160

gtg aac tcc ttc ctg gtc ttc atg gca tac aag gac cgg tgc cag tgc 528
Val Asn Ser Phe Leu Val Phe Met Ala Tyr Lys Asp Arg Cys Gln Cys
                165                 170                 175

agc gac agc cag atg tac gag atc ttc agc atc atc cgg gac ctg ggg 576
Ser Asp Ser Gln Met Tyr Glu Ile Phe Ser Ile Ile Arg Asp Leu Gly
            180                 185                 190

gcc ttg gcc cag gtg cac gct gag aac ggg gac atc gtg gag gag gag 624
Ala Leu Ala Gln Val His Ala Glu Asn Gly Asp Ile Val Glu Glu Glu
        195                 200                 205

cag aag cgg ttg ctg gag ctc ggc atc act ggc ccc gag ggc cac gtg 672
Gln Lys Arg Leu Leu Glu Leu Gly Ile Thr Gly Pro Glu Gly His Val
    210                 215                 220

ctc agc cac ccc gag gag gtg gag gct gag gcg gtg tac cga gct gtc 720
Leu Ser His Pro Glu Glu Val Glu Ala Glu Ala Val Tyr Arg Ala Val
225                 230                 235                 240

acc atc gcc aag cag gca aac tgc ccg ctg tac gtc acc aag gtg atg 768
Thr Ile Ala Lys Gln Ala Asn Cys Pro Leu Tyr Val Thr Lys Val Met
                245                 250                 255

agc aag ggg gcg gcc gac gcc atc gct cag gcc aag cgc aga ggg gtg 816
Ser Lys Gly Ala Ala Asp Ala Ile Ala Gln Ala Lys Arg Arg Gly Val
            260                 265                 270

gtc gtg ttt ggg gag ccc atc acc gcc agc ctg ggc acc gac ggt tca 864
Val Val Phe Gly Glu Pro Ile Thr Ala Ser Leu Gly Thr Asp Gly Ser
        275                 280                 285

cac tac tgg agc aag aac tgg gcc aag gcc gca gcc ttc gtc aca tca 912
His Tyr Trp Ser Lys Asn Trp Ala Lys Ala Ala Ala Phe Val Thr Ser
    290                 295                 300

ccc cct gtc aac cca gac ccc acc acg gca gac cac ctc acc tgc ttg 960
Pro Pro Val Asn Pro Asp Pro Thr Thr Ala Asp His Leu Thr Cys Leu
305                 310                 315                 320

ctg tcc agc ggg gac ctc cag gtg aca ggc agc gcc cac tgc acc ttc 1008
Leu Ser Ser Gly Asp Leu Gln Val Thr Gly Ser Ala His Cys Thr Phe
                325                 330                 335

acc act gcc cag aag gct gtg ggc aag gac aac ttc gcg ctg atc ccc 1056
Thr Thr Ala Gln Lys Ala Val Gly Lys Asp Asn Phe Ala Leu Ile Pro
            340                 345                 350

gag ggc acc aac ggc att gag gag cgc atg tcg atg gtc tgg gag aaa 1104
Glu Gly Thr Asn Gly Ile Glu Glu Arg Met Ser Met Val Trp Glu Lys
        355                 360                 365

tgt gtg gcc tct ggg aag atg gac gag aat gag ttc gtc gcg gtg acc 1152

Cys Val Ala Ser Gly Lys Met Asp Glu Asn Glu Phe Val Ala Val Thr
    370                 375                 380

agt aca aat gct gcc aaa atc ttc aat ttt tac cca agg aag ggg cga      1200
Ser Thr Asn Ala Ala Lys Ile Phe Asn Phe Tyr Pro Arg Lys Gly Arg
385                 390                 395                 400

gtg gct gtg ggc tct gac gct gac ctg gtc ata tgg aac ccc aag gcc      1248
Val Ala Val Gly Ser Asp Ala Asp Leu Val Ile Trp Asn Pro Lys Ala
                405                 410                 415

acc aag atc atc tct gcc aag acc cac aat ctg aac gtg gag tac aac      1296
Thr Lys Ile Ile Ser Ala Lys Thr His Asn Leu Asn Val Glu Tyr Asn
            420                 425                 430

atc ttc gag gga gtg gag tgc cgg gga gcg cct gcc gtg gtc ata agt      1344
Ile Phe Glu Gly Val Glu Cys Arg Gly Ala Pro Ala Val Val Ile Ser
        435                 440                 445

cag ggc cga gtg gcg ctg gag gac ggg aag atg ttt gtc acc ccg ggg      1392
Gln Gly Arg Val Ala Leu Glu Asp Gly Lys Met Phe Val Thr Pro Gly
    450                 455                 460

gcg ggc cgc ttc gtc cct cgg aaa aca ttc ccg gac ttt gtc tac aag      1440
Ala Gly Arg Phe Val Pro Arg Lys Thr Phe Pro Asp Phe Val Tyr Lys
465                 470                 475                 480

agg atc aaa gct cgc aac agg ctg gcg gag atc cac ggt gtg ccc cgt      1488
Arg Ile Lys Ala Arg Asn Arg Leu Ala Glu Ile His Gly Val Pro Arg
                485                 490                 495

ggg ctg tat gac ggg ccc gtc cac gag gtg atg gtg cct gcc aag cca      1536
Gly Leu Tyr Asp Gly Pro Val His Glu Val Met Val Pro Ala Lys Pro
            500                 505                 510

ggg agt ggc gct ccg gcc cgc gcg tcc tgc cca ggc aag atc tcc gtg      1584
Gly Ser Gly Ala Pro Ala Arg Ala Ser Cys Pro Gly Lys Ile Ser Val
        515                 520                 525

cct cct gtg cgc aac cta cat cag tcg ggg ttc agc cta tct ggg tct      1632
Pro Pro Val Arg Asn Leu His Gln Ser Gly Phe Ser Leu Ser Gly Ser
    530                 535                 540

cag gct gat gac cac atc gcc cga cgc aca gca cag aag atc atg gca      1680
Gln Ala Asp Asp His Ile Ala Arg Arg Thr Ala Gln Lys Ile Met Ala
545                 550                 555                 560

cca cct ggc ggc cgc tcc aac atc acc tct ctc tcc tag                  1719
Pro Pro Gly Gly Arg Ser Asn Ile Thr Ser Leu Ser
                565                 570

<210>  8
<211>  572
<212>  PRT
<213>  Homo sapiens

<400>  8

Met Ser Phe Gln Gly Lys Lys Ser Ile Pro Arg Ile Thr Ser Asp Arg
1               5                   10                  15

Leu Leu Ile Arg Gly Gly Arg Ile Val Asn Asp Asp Gln Ser Phe Tyr
            20                  25                  30

Ala Asp Val His Val Glu Asp Gly Leu Ile Lys Gln Ile Gly Glu Asn
        35                  40                  45

Leu Ile Val Pro Gly Gly Ile Lys Thr Ile Asp Ala His Gly Leu Met
    50                  55                  60

Val Leu Pro Gly Gly Val Asp Val His Thr Arg Leu Gln Met Pro Val
65                  70                  75                  80

Leu Gly Met Thr Pro Ala Asp Asp Phe Cys Gln Gly Thr Lys Ala Ala
                85                  90                  95

Leu Ala Gly Gly Thr Thr Met Ile Leu Asp His Val Phe Pro Asp Thr
            100                 105                 110

Gly Val Ser Leu Leu Ala Ala Tyr Glu Arg Trp Arg Glu Arg Ala Asp
        115                 120                 125

Ser Ala Ala Cys Cys Asp Tyr Ser Leu His Val Asp Ile Thr Arg Trp
    130                 135                 140

His Glu Ser Ile Lys Glu Glu Leu Glu Ala Leu Val Lys Glu Lys Gly
145                 150                 155                 160

Val Asn Ser Phe Leu Val Phe Met Ala Tyr Lys Asp Arg Cys Gln Cys
                165                 170                 175

Ser Asp Ser Gln Met Tyr Glu Ile Phe Ser Ile Ile Arg Asp Leu Gly
            180                 185                 190

Ala Leu Ala Gln Val His Ala Glu Asn Gly Asp Ile Val Glu Glu Glu
        195                 200                 205

Gln Lys Arg Leu Leu Glu Leu Gly Ile Thr Gly Pro Glu Gly His Val
    210                 215                 220

Leu Ser His Pro Glu Glu Val Glu Ala Glu Ala Val Tyr Arg Ala Val

225                 230                 235                 240

Thr Ile Ala Lys Gln Ala Asn Cys Pro Leu Tyr Val Thr Lys Val Met
                245                 250                 255

Ser Lys Gly Ala Ala Asp Ala Ile Ala Gln Ala Lys Arg Arg Gly Val
            260                 265                 270

Val Val Phe Gly Glu Pro Ile Thr Ala Ser Leu Gly Thr Asp Gly Ser
        275                 280                 285

His Tyr Trp Ser Lys Asn Trp Ala Lys Ala Ala Ala Phe Val Thr Ser
    290                 295                 300

Pro Pro Val Asn Pro Asp Pro Thr Thr Ala Asp His Leu Thr Cys Leu
305                 310                 315                 320

Leu Ser Ser Gly Asp Leu Gln Val Thr Gly Ser Ala His Cys Thr Phe
                325                 330                 335

Thr Thr Ala Gln Lys Ala Val Gly Lys Asp Asn Phe Ala Leu Ile Pro
            340                 345                 350

Glu Gly Thr Asn Gly Ile Glu Glu Arg Met Ser Met Val Trp Glu Lys
        355                 360                 365

Cys Val Ala Ser Gly Lys Met Asp Glu Asn Glu Phe Val Ala Val Thr
    370                 375                 380

Ser Thr Asn Ala Ala Lys Ile Phe Asn Phe Tyr Pro Arg Lys Gly Arg
385                 390                 395                 400

Val Ala Val Gly Ser Asp Ala Asp Leu Val Ile Trp Asn Pro Lys Ala
                405                 410                 415

Thr Lys Ile Ile Ser Ala Lys Thr His Asn Leu Asn Val Glu Tyr Asn
            420                 425                 430

Ile Phe Glu Gly Val Glu Cys Arg Gly Ala Pro Ala Val Val Ile Ser
        435                 440                 445

Gln Gly Arg Val Ala Leu Glu Asp Gly Lys Met Phe Val Thr Pro Gly
    450                 455                 460

Ala Gly Arg Phe Val Pro Arg Lys Thr Phe Pro Asp Phe Val Tyr Lys
465                 470                 475                 480

Arg Ile Lys Ala Arg Asn Arg Leu Ala Glu Ile His Gly Val Pro Arg
                485                 490                 495

Gly Leu Tyr Asp Gly Pro Val His Glu Val Met Val Pro Ala Lys Pro
            500                 505                 510

Gly Ser Gly Ala Pro Ala Arg Ala Ser Cys Pro Gly Lys Ile Ser Val
        515                 520                 525

Pro Pro Val Arg Asn Leu His Gln Ser Gly Phe Ser Leu Ser Gly Ser
    530                 535                 540

Gln Ala Asp Asp His Ile Ala Arg Arg Thr Ala Gln Lys Ile Met Ala
545                 550                 555                 560

Pro Pro Gly Gly Arg Ser Asn Ile Thr Ser Leu Ser
                565                 570

<210>  9
<211>  1713
<212>  DNA
<213>  Homo sapiens

<220>
<221>  CDS
<222>  (1)..(1713)
<223>  CRMP-4

<400>  9
atg tcc tac caa ggc aag aag aac atc ccg cgg atc acg agt gac cgt      48
Met Ser Tyr Gln Gly Lys Lys Asn Ile Pro Arg Ile Thr Ser Asp Arg
1               5                   10                  15

ctc ctt atc aag gga ggc aga atc gtc aat gat gat cag tcc ttt tat      96
Leu Leu Ile Lys Gly Gly Arg Ile Val Asn Asp Asp Gln Ser Phe Tyr
            20                  25                  30

gct gat att tac atg gaa gat ggc tta ata aaa caa att gga gac aat      144
Ala Asp Ile Tyr Met Glu Asp Gly Leu Ile Lys Gln Ile Gly Asp Asn
        35                  40                  45

ctg att gtt cct gga gga gtg aag acc att gaa gcc aat ggg aag atg      192
Leu Ile Val Pro Gly Gly Val Lys Thr Ile Glu Ala Asn Gly Lys Met
    50                  55                  60

gtg atc cct gga ggc atc gat gtc cat act cac ttc cag atg cca tat      240

Val Ile Pro Gly Gly Ile Asp Val His Thr His Phe Gln Met Pro Tyr
65                  70                  75                  80

aag gga atg acc aca gta gat gac ttc ttc caa ggg aca aag gcg gcc      288
Lys Gly Met Thr Thr Val Asp Asp Phe Phe Gln Gly Thr Lys Ala Ala
                85                  90                  95

tta gca ggt ggc acc acc atg atc att gac cat gtg gtg cct gag cct      336
Leu Ala Gly Gly Thr Thr Met Ile Ile Asp His Val Val Pro Glu Pro
            100                 105                 110

gag tcc agc ctg act gag gcc tat gag aaa tgg aga gag tgg gct gat      384
Glu Ser Ser Leu Thr Glu Ala Tyr Glu Lys Trp Arg Glu Trp Ala Asp
        115                 120                 125

ggg aag agt tgc tgt gac tat gcc ctg cat gtg gac atc acc cac tgg      432
Gly Lys Ser Cys Cys Asp Tyr Ala Leu His Val Asp Ile Thr His Trp
    130                 135                 140

aat gac agc gtc aag cag gaa gtg cag aac ctc atc aag gac aaa ggg      480
Asn Asp Ser Val Lys Gln Glu Val Gln Asn Leu Ile Lys Asp Lys Gly
145                 150                 155                 160

gtt aac tcc ttc atg gtt tat atg gct tat aag gat ttg tat caa gta      528
Val Asn Ser Phe Met Val Tyr Met Ala Tyr Lys Asp Leu Tyr Gln Val
                165                 170                 175

tct aac aca gag ctc tat gag atc ttc acc tgc ctg gga gag ctg ggg      576
Ser Asn Thr Glu Leu Tyr Glu Ile Phe Thr Cys Leu Gly Glu Leu Gly
            180                 185                 190

gcc att gct caa gtt cat gct gag aat ggg gat atc att gcc cag gag      624
Ala Ile Ala Gln Val His Ala Glu Asn Gly Asp Ile Ile Ala Gln Glu
        195                 200                 205

caa acc cgc atg ttg gaa atg ggg ata act ggc cca gaa ggc cat gta      672
Gln Thr Arg Met Leu Glu Met Gly Ile Thr Gly Pro Glu Gly His Val
    210                 215                 220

ctg agc agg cca gaa gag ctg gaa gct gag gct gtg ttc cgt gcc atc      720
Leu Ser Arg Pro Glu Glu Leu Glu Ala Glu Ala Val Phe Arg Ala Ile
225                 230                 235                 240

acc att gcc agc caa acc aat tgc cct ctc tac gtc aca aag gtc atg      768
Thr Ile Ala Ser Gln Thr Asn Cys Pro Leu Tyr Val Thr Lys Val Met
                245                 250                 255

agc aag agt gca gct gac ctc atc tca caa gcc agg aaa aaa gga aat      816
Ser Lys Ser Ala Ala Asp Leu Ile Ser Gln Ala Arg Lys Lys Gly Asn
            260                 265                 270

gta gtc ttt ggt gag ccc atc act gcc agc ctc ggc ata gat gga acc      864
Val Val Phe Gly Glu Pro Ile Thr Ala Ser Leu Gly Ile Asp Gly Thr
        275                 280                 285

cat tat tgg agc aag aac tgg gcc aag gcg gct gca ttt gtg aca tcc      912
His Tyr Trp Ser Lys Asn Trp Ala Lys Ala Ala Ala Phe Val Thr Ser
    290                 295                 300

cca ccc ctg agc cct gac cca act act ccg gac tac atc aac tcc ttg 960
Pro Pro Leu Ser Pro Asp Pro Thr Thr Pro Asp Tyr Ile Asn Ser Leu
305                 310                 315                 320

ctg gcc agc ggg gat ctg cag cta tct ggg agt gcc cac tgc acc ttc 1008
Leu Ala Ser Gly Asp Leu Gln Leu Ser Gly Ser Ala His Cys Thr Phe
                325                 330                 335

agc act gcc cag aaa gca att ggg aag gac aac ttc aca gcc att cct 1056
Ser Thr Ala Gln Lys Ala Ile Gly Lys Asp Asn Phe Thr Ala Ile Pro
            340                 345                 350

gag ggc acc aat ggt gtg gag gag cgg atg tct gtc atc tgg gac aag 1104
Glu Gly Thr Asn Gly Val Glu Glu Arg Met Ser Val Ile Trp Asp Lys
        355                 360                 365

gct gtg gcc aca ggg aaa atg gac gaa aac cag ttc gtg gct gtg aca 1152
Ala Val Ala Thr Gly Lys Met Asp Glu Asn Gln Phe Val Ala Val Thr
    370                 375                 380

agc aca aac gct gcc aag atc ttc aac ctg tat ccc cgc aag gga aga 1200
Ser Thr Asn Ala Ala Lys Ile Phe Asn Leu Tyr Pro Arg Lys Gly Arg
385                 390                 395                 400

ata tct gtg ggt tct gac agc gac ctc gtc atc tgg gat cca gat gct 1248
Ile Ser Val Gly Ser Asp Ser Asp Leu Val Ile Trp Asp Pro Asp Ala
                405                 410                 415

gtg aag atc gtc tct gcc aag aac cac cag tct gcg gca gag tac aac 1296
Val Lys Ile Val Ser Ala Lys Asn His Gln Ser Ala Ala Glu Tyr Asn
            420                 425                 430

atc ttt gaa ggg atg gag ctg cgc ggg gct cct ctg gtt gtc atc tgc 1344
Ile Phe Glu Gly Met Glu Leu Arg Gly Ala Pro Leu Val Val Ile Cys
        435                 440                 445

cag ggc aag atc atg ctg gaa gat ggc aac ctg cac gtg acc cag ggg 1392
Gln Gly Lys Ile Met Leu Glu Asp Gly Asn Leu His Val Thr Gln Gly
    450                 455                 460

gct ggc cgc ttc ata ccc tgc agc ccg ttc tcc gac tat gtc tac aag 1440
Ala Gly Arg Phe Ile Pro Cys Ser Pro Phe Ser Asp Tyr Val Tyr Lys
465                 470                 475                 480

cgc att aaa gca cgg agg aag atg gca gac ctg cat gcc gtc cca agg 1488
Arg Ile Lys Ala Arg Arg Lys Met Ala Asp Leu His Ala Val Pro Arg
                485                 490                 495

ggc atg tac gat ggg cct gtg ttt gac ctg acc acc acc ccc aaa ggt 1536
Gly Met Tyr Asp Gly Pro Val Phe Asp Leu Thr Thr Thr Pro Lys Gly
            500                 505                 510

ggc acc ccc gca ggc tct gct cgg ggc tct cct act cgg ccg aac cca 1584
Gly Thr Pro Ala Gly Ser Ala Arg Gly Ser Pro Thr Arg Pro Asn Pro
        515                 520                 525

cct gtg agg aat ctt cat cag tcg gga ttt agc ctg tca ggc acc caa 1632

Pro Val Arg Asn Leu His Gln Ser Gly Phe Ser Leu Ser Gly Thr Gln
    530                 535                 540

gtg gat gag ggg gtt cgc tca gcc agc aag cgc atc gtg gcg ccc cca      1680
Val Asp Glu Gly Val Arg Ser Ala Ser Lys Arg Ile Val Ala Pro Pro
545                 550                 555                 560

ggc ggc cgt tct aat atc aca tct ctg agt taa                          1713
Gly Gly Arg Ser Asn Ile Thr Ser Leu Ser
                565                 570

<210>  10
<211>  570
<212>  PRT
<213>  Homo sapiens

<400>  10

Met Ser Tyr Gln Gly Lys Lys Asn Ile Pro Arg Ile Thr Ser Asp Arg
1               5                   10                  15

Leu Leu Ile Lys Gly Gly Arg Ile Val Asn Asp Asp Gln Ser Phe Tyr
            20                  25                  30

Ala Asp Ile Tyr Met Glu Asp Gly Leu Ile Lys Gln Ile Gly Asp Asn
        35                  40                  45

Leu Ile Val Pro Gly Gly Val Lys Thr Ile Glu Ala Asn Gly Lys Met
    50                  55                  60

Val Ile Pro Gly Gly Ile Asp Val His Thr His Phe Gln Met Pro Tyr
65                  70                  75                  80

Lys Gly Met Thr Thr Val Asp Asp Phe Phe Gln Gly Thr Lys Ala Ala
                85                  90                  95

Leu Ala Gly Gly Thr Thr Met Ile Ile Asp His Val Val Pro Glu Pro
            100                 105                 110

Glu Ser Ser Leu Thr Glu Ala Tyr Glu Lys Trp Arg Glu Trp Ala Asp
        115                 120                 125

Gly Lys Ser Cys Cys Asp Tyr Ala Leu His Val Asp Ile Thr His Trp
    130                 135                 140

Asn Asp Ser Val Lys Gln Glu Val Gln Asn Leu Ile Lys Asp Lys Gly
145                 150                 155                 160

Val Asn Ser Phe Met Val Tyr Met Ala Tyr Lys Asp Leu' Tyr Gln Val
165 170 175

Ser Asn Thr Glu Leu Tyr Glu Ile Phe Thr Cys Leu Gly Glu Leu Gly
180 185 190

Ala Ile Ala Gln Val His Ala Glu Asn Gly Asp Ile Ile Ala Gln Glu
195 200 205

Gln Thr Arg Met Leu Glu Met Gly Ile Thr Gly Pro Glu Gly His Val
210 215 220

Leu Ser Arg Pro Glu Glu Leu Glu Ala Glu Ala Val Phe Arg Ala Ile
225 230 235 240

Thr Ile Ala Ser Gln Thr Asn Cys Pro Leu Tyr Val Thr Lys Val Met
245 250 255

Ser Lys Ser Ala Ala Asp Leu Ile Ser Gln Ala Arg Lys Lys Gly Asn
260 265 270

Val Val Phe Gly Glu Pro Ile Thr Ala Ser Leu Gly Ile Asp Gly Thr
275 280 285

His Tyr Trp Ser Lys Asn Trp Ala Lys Ala Ala Ala Phe Val Thr Ser
290 295 300

Pro Pro Leu Ser Pro Asp Pro Thr Thr Pro Asp Tyr Ile Asn Ser Leu
305 310 315 320

Leu Ala Ser Gly Asp Leu Gln Leu Ser Gly Ser Ala His Cys Thr Phe
325 330 335

Ser Thr Ala Gln Lys Ala Ile Gly Lys Asp Asn Phe Thr Ala Ile Pro
340 345 350

Glu Gly Thr Asn Gly Val Glu Glu Arg Met Ser Val Ile Trp Asp Lys
355 360 365

Ala Val Ala Thr Gly Lys Met Asp Glu Asn Gln Phe Val Ala Val Thr
370 375 380

Ser Thr Asn Ala Ala Lys Ile Phe Asn Leu Tyr Pro Arg Lys Gly Arg

385                 390                 395                 400

Ile Ser Val Gly Ser Asp Ser Asp Leu Val Ile Trp Asp Pro Asp Ala
                405                 410                 415

Val Lys Ile Val Ser Ala Lys Asn His Gln Ser Ala Ala Glu Tyr Asn
            420                 425                 430

Ile Phe Glu Gly Met Glu Leu Arg Gly Ala Pro Leu Val Val Ile Cys
        435                 440                 445

Gln Gly Lys Ile Met Leu Glu Asp Gly Asn Leu His Val Thr Gln Gly
    450                 455                 460

Ala Gly Arg Phe Ile Pro Cys Ser Pro Phe Ser Asp Tyr Val Tyr Lys
465                 470                 475                 480

Arg Ile Lys Ala Arg Arg Lys Met Ala Asp Leu His Ala Val Pro Arg
                485                 490                 495

Gly Met Tyr Asp Gly Pro Val Phe Asp Leu Thr Thr Thr Pro Lys Gly
            500                 505                 510

Gly Thr Pro Ala Gly Ser Ala Arg Gly Ser Pro Thr Arg Pro Asn Pro
        515                 520                 525

Pro Val Arg Asn Leu His Gln Ser Gly Phe Ser Leu Ser Gly Thr Gln
    530                 535                 540

Val Asp Glu Gly Val Arg Ser Ala Ser Lys Arg Ile Val Ala Pro Pro
545                 550                 555                 560

Gly Gly Arg Ser Asn Ile Thr Ser Leu Ser
                565                 570

<210>  11
<211>  1695
<212>  DNA
<213>  Homo sapiens

<220>
<221>  CDS
<222>  (1)..(1695)
<223>  CRMP-5

<400>  11
atg ctt gcc aac tca gcc agc gtg agg atc ctc atc aag gga ggc aag      48
Met Leu Ala Asn Ser Ala Ser Val Arg Ile Leu Ile Lys Gly Gly Lys
1               5                   10                  15

gtg gtg aac gat gac tgc acc cac gag gct gac gtc tac atc gag aat      96
Val Val Asn Asp Asp Cys Thr His Glu Ala Asp Val Tyr Ile Glu Asn
            20                  25                  30

ggc atc atc cag cag gtg ggc cgc gag ctc atg atc cct ggc ggg gcc      144
Gly Ile Ile Gln Gln Val Gly Arg Glu Leu Met Ile Pro Gly Gly Ala
        35                  40                  45

aag gtg att gat gcc aca gga aaa ctg gtg atc cct ggt ggc atc gac      192
Lys Val Ile Asp Ala Thr Gly Lys Leu Val Ile Pro Gly Gly Ile Asp
    50                  55                  60

acc agc acc cac ttc cac cag acc ttc atg aat gcc acg tgc gtg gac      240
Thr Ser Thr His Phe His Gln Thr Phe Met Asn Ala Thr Cys Val Asp
65                  70                  75                  80

gac ttc tac cat ggg acc aag gca gca ctc gtc gga ggc acc acc atg      288
Asp Phe Tyr His Gly Thr Lys Ala Ala Leu Val Gly Gly Thr Thr Met
                85                  90                  95

atc atc ggc cac gtc ctg ccc gac aag gag acc tcc ctt gtg gac gct      336
Ile Ile Gly His Val Leu Pro Asp Lys Glu Thr Ser Leu Val Asp Ala
            100                 105                 110

tat gag aag tgc cga ggt ctg gcc gac ccc aag gtc tgc tgt gat tac      384
Tyr Glu Lys Cys Arg Gly Leu Ala Asp Pro Lys Val Cys Cys Asp Tyr
        115                 120                 125

gcc ctc cac gtg ggg atc acc tgg tgg gca ccc aag gtg aaa gca gaa      432
Ala Leu His Val Gly Ile Thr Trp Trp Ala Pro Lys Val Lys Ala Glu
    130                 135                 140

atg gag aca ctg gtg agg gag aag ggt gtc aac tcg ttc cag atg ttc      480
Met Glu Thr Leu Val Arg Glu Lys Gly Val Asn Ser Phe Gln Met Phe
145                 150                 155                 160

atg acc tac aag gac ctg tac atg ctt cga gac agt gag ctg tac caa      528
Met Thr Tyr Lys Asp Leu Tyr Met Leu Arg Asp Ser Glu Leu Tyr Gln
                165                 170                 175

gtg ttg cac gct tgc aag gac att ggg gca atc gcc cgc gtc cat gct      576
Val Leu His Ala Cys Lys Asp Ile Gly Ala Ile Ala Arg Val His Ala
            180                 185                 190

gaa aat ggg gag ctt gtg gcc gag ggt gct aag gag gca ctg gat ttg      624
Glu Asn Gly Glu Leu Val Ala Glu Gly Ala Lys Glu Ala Leu Asp Leu
        195                 200                 205

ggg atc aca ggc cca gaa gga atc gag atc agc cgt cca gag gag ctg      672
Gly Ile Thr Gly Pro Glu Gly Ile Glu Ile Ser Arg Pro Glu Glu Leu
    210                 215                 220

gaa gct gaa gcc act cat cgt gtt atc acc att gca aac agg act cac      720

Glu Ala Glu Ala Thr His Arg Val Ile Thr Ile Ala Asn Arg Thr His
225                 230                 235                 240

tgt cca atc tac ctg gtc aac gtg tcc agt atc tcg gct ggt gac gtt      768
Cys Pro Ile Tyr Leu Val Asn Val Ser Ser Ile Ser Ala Gly Asp Val
                245                 250                 255

atc gca gct gct aag atg caa ggg aag gtt gtg ctg gcg gag acc acc      816
Ile Ala Ala Ala Lys Met Gln Gly Lys Val Val Leu Ala Glu Thr Thr
            260                 265                 270

act gca cat gcc acg ctg aca ggc tta cac tac tac cac cag gac tgg      864
Thr Ala His Ala Thr Leu Thr Gly Leu His Tyr Tyr His Gln Asp Trp
        275                 280                 285

tcc cac gcg gct gcc tat gtc acg gtg cct ccc ctg aga ctg gac acc      912
Ser His Ala Ala Ala Tyr Val Thr Val Pro Pro Leu Arg Leu Asp Thr
    290                 295                 300

aac acc tca acc tac ctc atg agc ctg ctg gcc aat gac act ctg aac      960
Asn Thr Ser Thr Tyr Leu Met Ser Leu Leu Ala Asn Asp Thr Leu Asn
305                 310                 315                 320

atc gtg gca tca gat cac cgg cct ttc acc aca aag cag aaa gct atg     1008
Ile Val Ala Ser Asp His Arg Pro Phe Thr Thr Lys Gln Lys Ala Met
                325                 330                 335

ggc aag gaa gac ttc acc aag atc cca cat gga gtg agt ggc gtg cag     1056
Gly Lys Glu Asp Phe Thr Lys Ile Pro His Gly Val Ser Gly Val Gln
            340                 345                 350

gac cgc atg agc gtc atc tgg gag aga gga gtg gtt gga gga aag atg     1104
Asp Arg Met Ser Val Ile Trp Glu Arg Gly Val Val Gly Gly Lys Met
        355                 360                 365

gat gag aac cgt ttt gtg gcc gtt acc agt tcc aac gca gct aag ctt     1152
Asp Glu Asn Arg Phe Val Ala Val Thr Ser Ser Asn Ala Ala Lys Leu
    370                 375                 380

ctg aac ctg tat ccc cgc aag ggc cgc att att ccc gga gcc gat gct     1200
Leu Asn Leu Tyr Pro Arg Lys Gly Arg Ile Ile Pro Gly Ala Asp Ala
385                 390                 395                 400

gat gtg gtg gtg tgg gac cca gaa gcc aca aag acc atc tca gcc agc     1248
Asp Val Val Val Trp Asp Pro Glu Ala Thr Lys Thr Ile Ser Ala Ser
                405                 410                 415

acg cag gtc cag gga gga gac ttc aac ctg tat gag aac atg cgc tgc     1296
Thr Gln Val Gln Gly Gly Asp Phe Asn Leu Tyr Glu Asn Met Arg Cys
            420                 425                 430

cac ggc gtg cca ctg gtc acc atc agc cgg ggg cgc gtc gtg tat gag     1344
His Gly Val Pro Leu Val Thr Ile Ser Arg Gly Arg Val Val Tyr Glu
        435                 440                 445

aac ggc gtc ttc atg tgc gcc gag ggc acc ggc aag ttc tgt ccc ctg     1392
Asn Gly Val Phe Met Cys Ala Glu Gly Thr Gly Lys Phe Cys Pro Leu
    450                 455                 460

agg tcc ttc cca gac act gtc tac aag aag ctg gtc cag aga gag aag     1440
Arg Ser Phe Pro Asp Thr Val Tyr Lys Lys Leu Val Gln Arg Glu Lys
465                 470                 475                 480

act tta aag gtt aga gga gtg gac cgc act ccc tac ctg ggg gat gtc     1488
Thr Leu Lys Val Arg Gly Val Asp Arg Thr Pro Tyr Leu Gly Asp Val
                485                 490                 495

gct gtt gtc gtg cac cct ggg aaa aaa gag atg gga acc cca ctc gca     1536
Ala Val Val Val His Pro Gly Lys Lys Glu Met Gly Thr Pro Leu Ala
            500                 505                 510

gac act cct acc cgg ccc gtc acc cgg cat ggg ggc atg agg gac ctt     1584
Asp Thr Pro Thr Arg Pro Val Thr Arg His Gly Gly Met Arg Asp Leu
        515                 520                 525

cac gaa tcc agc ttc agc ctc tct ggc tct cag atc gat gac cat gtt     1632
His Glu Ser Ser Phe Ser Leu Ser Gly Ser Gln Ile Asp Asp His Val
    530                 535                 540

cca aag cga gct tca gct cgg atc ctc gct cct ccc gga ggc agg tcg     1680
Pro Lys Arg Ala Ser Ala Arg Ile Leu Ala Pro Pro Gly Gly Arg Ser
545                 550                 555                 560

agt ggc att tgg taa                                                 1695
Ser Gly Ile Trp

<210>  12
<211>  564
<212>  PRT
<213>  Homo sapiens

<400>  12

Met Leu Ala Asn Ser Ala Ser Val Arg Ile Leu Ile Lys Gly Gly Lys
1               5                   10                  15

Val Val Asn Asp Asp Cys Thr His Glu Ala Asp Val Tyr Ile Glu Asn
            20                  25                  30

Gly Ile Ile Gln Gln Val Gly Arg Glu Leu Met Ile Pro Gly Gly Ala
        35                  40                  45

Lys Val Ile Asp Ala Thr Gly Lys Leu Val Ile Pro Gly Gly Ile Asp
    50                  55                  60

Thr Ser Thr His Phe His Gln Thr Phe Met Asn Ala Thr Cys Val Asp
65                  70                  75                  80

Asp Phe Tyr His Gly Thr Lys Ala Ala Leu Val Gly Gly Thr Thr Met

                85                  90                  95

Ile Ile Gly His Val Leu Pro Asp Lys Glu Thr Ser Leu Val Asp Ala
            100                 105                 110

Tyr Glu Lys Cys Arg Gly Leu Ala Asp Pro Lys Val Cys Cys Asp Tyr
        115                 120                 125

Ala Leu His Val Gly Ile Thr Trp Trp Ala Pro Lys Val Lys Ala Glu
    130                 135                 140

Met Glu Thr Leu Val Arg Glu Lys Gly Val Asn Ser Phe Gln Met Phe
145                 150                 155                 160

Met Thr Tyr Lys Asp Leu Tyr Met Leu Arg Asp Ser Glu Leu Tyr Gln
                165                 170                 175

Val Leu His Ala Cys Lys Asp Ile Gly Ala Ile Ala Arg Val His Ala
            180                 185                 190

Glu Asn Gly Glu Leu Val Ala Glu Gly Ala Lys Glu Ala Leu Asp Leu
        195                 200                 205

Gly Ile Thr Gly Pro Glu Gly Ile Glu Ile Ser Arg Pro Glu Glu Leu
    210                 215                 220

Glu Ala Glu Ala Thr His Arg Val Ile Thr Ile Ala Asn Arg Thr His
225                 230                 235                 240

Cys Pro Ile Tyr Leu Val Asn Val Ser Ser Ile Ser Ala Gly Asp Val
                245                 250                 255

Ile Ala Ala Ala Lys Met Gln Gly Lys Val Val Leu Ala Glu Thr Thr
            260                 265                 270

Thr Ala His Ala Thr Leu Thr Gly Leu His Tyr Tyr His Gln Asp Trp
        275                 280                 285

Ser His Ala Ala Ala Tyr Val Thr Val Pro Pro Leu Arg Leu Asp Thr
    290                 295                 300

Asn Thr Ser Thr Tyr Leu Met Ser Leu Leu Ala Asn Asp Thr Leu Asn
305                 310                 315                 320

Ile Val Ala Ser Asp His Arg Pro Phe Thr Thr Lys Gln Lys Ala Met
                325                 330                 335

Gly Lys Glu Asp Phe Thr Lys Ile Pro His Gly Val Ser Gly Val Gln
            340                 345                 350

Asp Arg Met Ser Val Ile Trp Glu Arg Gly Val Val Gly Gly Lys Met
        355                 360                 365

Asp Glu Asn Arg Phe Val Ala Val Thr Ser Ser Asn Ala Ala Lys Leu
    370                 375                 380

Leu Asn Leu Tyr Pro Arg Lys Gly Arg Ile Ile Pro Gly Ala Asp Ala
385                 390                 395                 400

Asp Val Val Val Trp Asp Pro Glu Ala Thr Lys Thr Ile Ser Ala Ser
                405                 410                 415

Thr Gln Val Gln Gly Gly Asp Phe Asn Leu Tyr Glu Asn Met Arg Cys
            420                 425                 430

His Gly Val Pro Leu Val Thr Ile Ser Arg Gly Arg Val Val Tyr Glu
        435                 440                 445

Asn Gly Val Phe Met Cys Ala Glu Gly Thr Gly Lys Phe Cys Pro Leu
    450                 455                 460

Arg Ser Phe Pro Asp Thr Val Tyr Lys Lys Leu Val Gln Arg Glu Lys
465                 470                 475                 480

Thr Leu Lys Val Arg Gly Val Asp Arg Thr Pro Tyr Leu Gly Asp Val
                485                 490                 495

Ala Val Val Val His Pro Gly Lys Lys Glu Met Gly Thr Pro Leu Ala
            500                 505                 510

Asp Thr Pro Thr Arg Pro Val Thr Arg His Gly Gly Met Arg Asp Leu
        515                 520                 525

His Glu Ser Ser Phe Ser Leu Ser Gly Ser Gln Ile Asp Asp His Val
    530                 535                 540

Pro Lys Arg Ala Ser Ala Arg Ile Leu Ala Pro Pro Gly Gly Arg Ser

545                 550                 555                 560

Ser Gly Ile Trp

## Description

The present invention refers to a method for identifying a modulator of CRMP suitable for the prevention, alleviation or/and treatment of CRMP associated diseases. The present invention further refers to a cell capable of overexpressing or underexpressing CRMP, and to a non-human animal comprising such cell. Further subjects of the present invention are compounds suitable for the prevention, alleviation or/and treatment of CRMP-associated diseases.

Charrier et al. (Molecular Neurobiology, 2003, 28:51-63) describe the family of collapsin response mediator proteins (CRMP-1 to 5) as developmentally regulated proteins strongly expressed in postmitotic neural cells in the developing nervous system. In the adult brain, the CRMPs are dramatically downregulated.

CRMP-2 (also called CRMP-62; Dihydropyrimidinase-related protein 2, DRP-2; TOAD-64; Ulip2) is described in the literature as being involved in the regulation of neuronal polarity. CRMP-2 binds to tubulin heterodimers and promotes microtubule assembly. CRMP-2/tubulin complexes modulate microtubule dynamics and contribute to axonal growth.

In hippocampal neurons, CRMP-2 accumulates in the distal part of the growing axons. Overexpression of CRMP-2 induces the formation of multiple axons, in contrast to axons normally having a single axon and several dendrites. Overexpression of CRMP-2 promotes axonal growth and branching. CRMP-2 blockade inhibits axon formation and CRMP-2 inhibition attenuates neurotrophic factor induced axon propagation (NT3/BDNF). Dominant negative mutants of CRMP-2 (truncated mutants) suppress axonal growth (Arimura N. et al., J. Neurobiol. 2004, 58:34-47).

Axogenesis and spatial and temporal dendrite formation have serious effects on the function of the nervous system. Disruptions of neuronal polarity are frequently associated with mental illness such as neurodegenerative diseases, e.g. Alzheimer's disease.

Nakata et al. (Biol. Psychiatry 2003, 53:571-576) discuss the role of CRMP-2 polymorphism in paranoid-type schizophrenia on the basis of allele frequencies of CRMP-2 gene polymorphism. The correlation suggests that a specific mutation may reduce the susceptibility to schizophrenia, especially to the paranoid subtype. There is no hint that CRMP-2 would be a target for therapy of schizophrenia, e.g. in combination with antipsychotic drugs.

In Down syndrome and Alzheimer patients, deranged CRMP-2 levels may contribute to the abnormal plasticity and wiring observed in the brains of these patients (Nakata et al., Biol Psychiatry 2003; 53:571-576).

In hypothyroidism, which is characterized by abnormalities of central nervous system development, CRMP-2 was found to be dysregulated (Charrier et al., Mol. Neurobiology 2003; 28(1): 51-63).

In Parkinson's disease, CRMP-2 could be involved in dopamine-induced neuronal apoptosis, possibly via semaphorin 3A (Charrier et al., Mol. Neurobiology 2003; 28(1): 51-63). There is, however, no indication that CRMP-2 would be a target for the treatment of Parkinsonian tremor syndromes.

CRMP-2 was shown to interact directly with PLD₂ (Lee et al., J. Biol. Chem. 2002, 277:6542-6549) by amino acids 243-300 (rat CRMP-2). CRMP-2 was found to inhibit PLD₂ activity concentration dependently in COS-7 cells. CRMP-2 was found to be co-localized in the distal tips of neurites in differentiated PC12 cells. In PC12 cells, semaphorin 3A inhibits PLD₂ activity, indicating the CRMP-2 may be an intracellular mediator of semaphorin 3A.

Phosphorylation of CRMP-2 seems to be the major regulatory mechanism of CRMP-2 activity. Phosphorylated CRMP-2 is inactive; dephosphorylated CRMP-2 regulates microtubule assembly in axons by regulation of the polymerization rate by the tubulin dimer pool and stabilization of the microtubule polymer (Nakata et al., Biol Psychiatry 2003; 53:571-576). Glycogen synthase kinase-3β (GSK-3β) was shown to phosphorylate CRMP-2 at Thr-514, thus inactivating it and thereby regulating neurotrophin-induced axonal outgrowth (Yoshimura et al, Cell 2005; 120: 137-149).

Apart from its function in neurodevelopment, CRMP-2 was shown to be involved in pathways that regulate the proliferation of non-neuronal cells through its phosphorylation by regulatory proteins. It was suggested that deregulation of CRMP-2 phosphorylation levels may lead to aberrant cell proliferation and consequently, tumorgenesis (Tahimic et al, Biochem. Biophys. Res. Commun. 2006; 340:1244-1250).

Czech et al. (Neurochemical Research 2004, 29:2189-2196) describe reduction of CRMP-2 in patients suffering from mesial temporal lobe epilepy (MTLE).

The CRMP-2 family of proteins is implicated in developmental process of the nervous system since most of the five CRMP proteins are highly expressed during early development and mainly in the central nervous system. On an in-vitro level, CRMP-2 has been shown to be involved in neuronal differentiation, polarization and axonal outgrowth induced by neurotrophic factors such as brain derived neurotrophic factor (BDNF). Interestingly rearrangement of neuronal connections and erroneous neuronal outgrowth has been implicated in the pathophysiology of epilepsy and neuropathic pain (Pezet, McMchon, Ann. Rev. Neurosci 2006, 29:507-38, Binder 2004, Adv. Exp. Med. Biol. 548:34-56). Moreover, BNDF is involved in the develoment of epilepsy and chronic pain in animal models.

Lacosamide (international non-proprietory name) is known to be an anticonvulsant and analgesic drug. Lacosamide belongs to a class of peptidic compounds known to exhibit central nervous system activity. These peptides are described in the U.S. Patent Nos. 5,378,729 and 5,773,475. In the context of the present invention, these compounds are referred to as Lacosamide-related compounds.

The standard chemical nomenclature of Lacosamide is (R)-2-acetamide-N-benzyl-3-methoxypropionamide. Lacosamide is also known as SPM 927 or Harkoseride.

Lacosamide is in phase III clinical development in the indications epilepsy and diabetic neuropathy pain. Preclinical data support a potential of Lacosamide in the treatment of the following pain syndromes: diabetic neuropathy, chemotherapy-induced neuropathy, fibromyalgia, osteoarthritis, cancer pain, nerve lesion-induced pain, inflammatory pain and central pain. Additional preclinical data support a potential of Lacosamide in the treatment of the following disease areas: essential tremor, dyskinesias, migraine prophylaxis, amyotrophic lateral sclerosis and psychosis, especially schizophrenia. Finally, Lacosamide has shown preclinical and clinical efficacy for the treatment of complex partial seizures with and without secondary generalization, and *status epilepticus.*

The mechanism of action of anticonvulsants in disease treatment is only beginning to be understood. The broad activity of Lacosamide is described in various disease models, although the molecular mode of action for these effects remains unknown.

It was therefore the object of the present invention to provide a method of identifying compounds suitable for the prevention, alleviation or/and treatment of diseases or conditions which can be treated by Lacosamide, based on the mode of action of Lacosamide. Surprisingly, in protein binding experiments, it was found that Lacosamide binds directly to CRMP-2 in extracts obtained from rat brain. The binding was confirmed in experiments employing heterologously expressed human CRMP-2 in Xenopus oocytes. A functional interaction of CRMP-2 and Lacosamide was shown in experiments exploring the effect of Lacosamide on the neurotrophin-induced stimulation of axonal outgrowth in cultured hippocampal neurons. CRMP-2 is therefore regarded as a target of Lacosamide in the nervous system in treatment of epilepsy, pain, essential tremor, dyskinesias, amyotrophic lateral sclerosis, schizophrenia and other diseases or conditions as described herein.

This object is achieved according to the invention by a method for identifying a modulator of CRMP suitable for the prevention, alleviation or/and treatment of a CRMP associated disease, comprising the steps
(a) selecting a substance,
(b) contacting the substance selected in step (a) with CRMP, and
(c) determining whether CRMP interacts with the substance,
wherein a substance interacting with CRMP is a modulator of CRMP, in particular a modulator of CRMP suitable for the prevention, alleviation or/and treatment of a CRMP associated disease.

The CRMP associated disease of the present invention may be a disease which can by prevented, alleviated or/and treated by Lacosamide or a Lacosamide-related compound.

A preferred CRMP associated disease is a CRMP-2 associated disease.

It is preferred that the CRMP associated disease, in particular the CRMP-2 associated disease is a neurological disease, such as epilepsy, a pain syndrome, a motoneuron disorder, a dyskinesia, or a tremor syndrome. "Neurological disease" also includes arthritic conditions like osteoarthritis, rheumatoid arthritis including juvenile rheumatoid arthritis, fibromyalgia, and musculoskeletal disorders as defined herein.

It is also preferred that the CRMP associated disease, in particular the CRMP-2 associated disease, is a psychiatric disease, such as psychosis or/and schizophrenia.

It is also preferred that the CRMP associated disease, in particular the CRMP-2 associated disease, is an inflammatory disease such as arthritis or an arthritic condition associated with inflammation, e.g. inflammatory osteoarthritis.

Thus, the CRMP associated disease, in particular the CRMP-2 associated disease, is preferably selected from neurological diseases, psychiatric diseases, or/and inflammatory diseases.

It is also preferred that the CRMP-associated disease, in particular the CRMP-2 associated disease, of the present invention is selected from epilepsy, pain syndromes, motoneuron disorders, dyskinesias, tremor syndromes, psychosis, especially schizophrenia, arthritis or an arthritic condition such as fibromyalgia and osteoarthritis, any condition or disease included therein as described herein, and combinations thereof.

It is more preferred that the CRMP-associated disease of the present invention, in particular the CRMP-2 associated disease, is selected from epilepsy, pain syndromes, motoneuron disorders, dyskinesias, tremor syndromes different from Parkinsonian tremor syndrome, arthritis or an arthritic condition such as fibromyalgia and osteoarthritis, any condition or disease included therein as described herein, and combinations thereof.

It is even more preferred that the CRMP-associated disease of the present invention, in particular the CRMP-2 associated disease, is selected from epilepsy, pain syndromes, arthritis or an arthritic condition such as fibromyalgia and osteoarthritis, any condition or disease included therein as described herein, and combinations thereof. Preferred epilepsy conditions are *status epilepticus* and complex partial seizures with and without secondary generalization. A preferred pain syndrome is painful diabetic neuropathy, preferably associated with Diabetes mellitus Type I or II, more preferably Type II. Another preferred pain syndrome is pain associated with arthritis or an arthritic condition, in particular with osteoarthritis.

Epilepsy includes, but is not limited to, complex partial seizures with and without secondary generalization, *status epilepticus* and a *status epilepticus-*related condition, e.g. acute repetitive seizures, seizure clusters, etc. Further, epilepsy, in particular before/during acute seizures, may require neuroprotective treatment to reduce brain damage, short term memory loss, cognitive decline, or/and additional seizures (anti-epileptogenesis). *Status epilepticus* includes partial or/and generalized seizures. Generalized seizures can be convulsive, such as tonic-clonic, tonic, clonic, or myoclonic seizures, or non-convulsive, such as absences or atonic seizures. Details of the prevention, alleviation or/and treatment of *status epilepticus* and neuroprotective treatment by Lacosamide are described in EP 1 541 138, the disclosure of which is incorporated herein by reference.

Further, epilepsy includes a refractory epileptic condition. The term "refractory epileptic condition" herein refers to an epileptic disease state such as *status epilepticus,* an epileptic seizure, a repetitive seizure or a seizure cluster that is at least partially or substantially resistant to treatment with one or more anti-epileptic drugs. The term "refractory *status epilepticus"* herein refers to *status epilepticus* as defined above exhibiting at least partial or substantial resistance to treatment with one or more anti-epileptic drugs. Such drugs in either case include benzodiazepines, barbiturates and anticonvulsants other than a compound of Formula (I) as defined herein. For example and without limitation, resistance can be exhibited to treatment with one or more drugs selected from diazepam, lorazepam, midazolam, phenobarbital, carbamazepine, phenytoin, fosphenytoin, oxcarbazepine, lamotrigine, gabapentin, pregabalin, valproic acid, pentobarbital, thiopental, propofol and pharmaceutically acceptable salts thereof.

Further, refractory *status epilepticus* as used herein may be initially responsive to treatment with such drugs but becomes at least partially refractory when it lasts for at least about 10 minutes, for example at least about 15 minutes, at least about 20 minutes, at least about 30 minutes, at least about 45 minutes or at least about 60 minutes.

Further, a refractory epileptic condition including refractory *status epilepticus* can be present a *priori,* or, in the case of refractory *status epilepticus,* can be associated with the duration of *status epilepticus* as indicated above.

It is preferred that epilepsy as used herein is not mesial temporal lobe epilepsy (MTLE).

Pain syndromes include, but are not limited to, allodynia, phantom pain, acute and chronic pain, neuropathic pain including central neuropathic pain and peripheral neuropathic pain, painful diabetic neuropathy, painful conditions associated with or/and caused by cortical spreading depression (CSD), pain associated with a mononeuropathy, tumor pain, chemotherapy induced pain, nucleoside induced pain, and nucleoside analogue induced pain, non-inflammatory musculoskeletal pain, pain associated with arthritis or with an arthritic condition.

Allodynia includes, but is not limited to, allodynia as a major and unique pain symptom independent of the nature of the underlying disease, and phantom pain. Details of the prevention, alleviation or/and treatment of allodynia by Lacosamide are described in EP 1 243 263, the disclosure of which is incorporated herein by reference.

Details of the prevention, alleviation or/and treatment of phantom pain by the CRMP modulator Lacosamide are described in EP 1 243 263, the disclosure of which is incorporated herein by reference.

Acute and chronic pain include, but are not limited to, non neuropathic inflammatory pain including chronic inflammatory pain, rheumatoid arthritis pain, and secondary inflammatory osteoarthritic pain. Details of the prevention, alleviation or/and treatment of acute and chronic pain by Lacosamide are described in EP 1 243 262, the disclosure of which is incorporated herein by reference.

Neuropathic pain includes, but is not limited to, pain associated with lesions of the nervous system. Neuropathic pain includes peripheral and central neuropathic pain.

Central neuropathic pain includes, but is not limited to, spinal cord injury pain or/and CNS injury pain. Details of the prevention, alleviation or/and treatment of central neuropathic pain by Lacosamide are described in WO 2005/053667 A1, the disclosure of which is incorporated herein by reference.

Peripheral neuropathic pain includes, but is not limited to, pain associated with injury, infection or dysfunction of peripheral sensory nerves.

Painful diabetic neuropathy includes, but is not limited to, a condition associated with painful diabetic neuropathy, such as, but not limited to, average daily pain, overall pain, present pain intensity, pain interference with sleep, the subject's perception of pain interference with general activity, the patients' global impression of change in pain, clinical global impression of change in pain, the subject's perception of different neuropathic pain qualities, quality of life and proportion of pain-free days. A preferred painful diabetic neuropathy is a diabetic distal sensory polyneuropathy. The painful diabetic neuropathy may be associated with Diabetes mellitus Type I or Diabetes mellitus Type II. Details of the prevention, alleviation or/and treatment of painful diabetic neuropathy by Lacosamide are described in WO 2005/092313 A1, the disclosure of which is incorporated herein by reference.

Painful conditions associated with or/and caused by cortical spreading depression (CSD) include, but are not limited to, chronic headache, cerebral ischemia during stroke or cardiovascular surgery, traumatic brain injury, subarachnoid hemorrhage or transient global amnesia. The chronic headache associated with or/and caused by CSD may be selected from migraine and other forms of chronic headache of both central and peripheral origin such as, but not limited to, cluster headache, tension-type headache, secondary headaches associated with over use of medication, cranial neuralgias, brain trauma and vascular or metabolic disorders. The migraine is preferably acute migraine. Details of the prevention, alleviation or/and treatment of painful conditions associated with or/and caused by CSD, in particular chronic headache, with Lacosamide are described in WO 2005/099740 A1, the disclosure of which is incorporated herein by reference.

Pain associated with a mononeuropathy includes, but is not limited to, trigeminal neuropathic pain, pain associated with trigeminal neuralgia, and atypical facial pain. Details of the prevention, alleviation or/and treatment of trigeminal neuropathic pain by Lacosamide are described in WO 2005/120539 A2, the disclosure of which is incorporated herein by reference.

Tumor pain includes, but is not limited to, tumor pain associated with AIDS, bone cancer pain, pain produced during tumor progression by infiltration in or pressure on bone, viscera, soft tissue or/and nerves, metastasis-induced pain, metastasis-induced bone cancer pain or/and pain induced by metastatic disease in patients with breast, prostate or/and lung cancer.

Chemotherapy-induced pain includes, but is not limited to, chemotherapy-induced neuropathic pain, vinca alkaloid-induced pain or/and vincristine-induced pain.

Nucleoside or/and nucleoside analogue-induced pain include, but are not limited to, painful peripheral neuropathy induced by nucleosides or/and nucleoside analogues, pain induced by anti-tumor or/and anti-viral nucleoside analogues, pain induced by anti-viral nucleoside analogues in AIDS therapy, and pain induced by AZT, ddC, ddl or/and d4T.

Details of the prevention, alleviation or/and treatment of tumor pain, chemotherapy induced pain, nucleoside induced pain and nucleoside analogue induced pain by Lacosamide are described in WO 2006/021412 A2, the disclosure of which is incorporated herein by reference.

Details of the prevention, alleviation or/and treatment of non-inflammatory musculoskeletal pain, in particular specific manifestations of non-inflammatory musculoskeletal pain such as muscular hyperalgesia or/and allodynia occurring in fibromyalgia, myofascial pain syndrome or/and back pain are described in the unpublished application EP 05 017 979.9, which is included herein by reference.

In the present invention, non-inflammatory musculoskeletal pain includes, but is not limited to non-inflammatory musculoskeletal pain associated with or/and caused by a pathological condition. Preferably, this condition is selected form regional pain syndrome such as back or neck pain, rheumatoid arthritis, osteoarthritis, gout, ankylosing spondylitis, lupus erythematosus, fibromyalgia, fibrositis, fibromyositis, myofascial pain syndrome, autoimmune disorders, polymyalgia rheumatica, polymyositis, dermatomyositis, muscular abscess, trichinosis, Lyme disease, Malaria, Rocky Mountain spotted fever, and polio.

Non-inflammatory musculoskeletal pain may be responsible for a number of symptoms, which may be remedied or at least relieved by the treatment of the non-inflammatory musculoskeletal pain. Therefore, in the present invention, non-inflammatory musculoskeletal pain includes, but is not limited to a condition associated with or/and caused by non-inflammatory musculoskeletal pain. Preferably, this condition is selected from fatigue, sleep disorder, irritable bowel syndrome, chronic headache, temporo-mandibular joint dysfunction syndrome, multiple chemical sensitivity, painful menstrual periods, dysmenorrhea, chest pain, morning stiffness, cognitive or memory impairment, numbness and tingling sensations, muscle twitching, irritable bladder, the feeling of swollen extremities, skin sensitivities, dry eyes and mouth, frequent changes in eye prescription, dizziness and impaired coordination.

Pain associated with arthritis or/and pain associated with an arthritic condition includes, but is not limited to osteoarthritic pain such as inflammatory osteoarthritic pain and non-infammatory osteoarthritic pain, pain associated with rheumatoid arthritis including juvenile rheumatoid arthritis, fibromyalgia pain and pain associated with a musculoskeletal disorder of one or more joints of a subject including arthritis associated with or secondary to conditions not necessary primarily arthritic. An arthritic condition includes, but is not limited to psoriatic arthritis, infectious arthritis, ankylosing spondylitis, neurogenic arthropathy and polyarthralgia. Conditions to which arthritis can be secondary include without limitation Sjögren's syndrome, Behçet's syndrome, Reiter's syndrome, systemic lupus erythematosus, rheumatic fever, gout, pseudogout, Lyme disease, sarcoidosis and ulcerative colitis.

The definition of pain associated with arthritis or with an arthritic condition overlaps with non-inflammatory musculoskeletal pain as defined herein.

Motoneuron disorders include, but are not limited to, amyotrophic lateral sclerosis (ALS), progressive spinal muscular atrophy, progressive bulbar palsy, and peripheral neuropathies, such as, but not limited to, Guillain-Barré Syndrome and Charcot-Marie-Tooth Syndrome. Details of the prevention, alleviation or/and treatment of motoneuron disorders such as ALS by Lacosamide are described in WO 2005/120476 A2, the disclosure of which is incorporated herein by reference.

Dyskinesias include, but are not limited to, primary dyskinesias such as, but not limited to, Huntington's chorea and cerebral palsy, and secondary dyskinesias such as, but not limited to, tardive and L-DOPA-induced dyskinesia. The dyskinesia forms include chorea, athetosis, dystonia, ballismus, and combinations thereof. Details of the prevention, alleviation or/and treatment of dyskinesias by Lacosamide are described in WO 2005/110390, the disclosure of which is incorporated herein by reference.

Tremor includes, but is not limited to, essential tremor, physiologic tremor, enhanced physiologic tremor, undetermined tremor syndrome, primary orthostatic tremor, dystonic tremor, task- and position-specific tremors, Parkinsonian tremor syndromes, cerebellar tremor syndromes, Holmes tremor, palatal tremors, neuropathic tremor syndrome, drug-induced and toxic tremor syndromes, psychogenic tremor, myorhythmia, rest tremor, action tremor, postural tremor, kinetic tremor, task- or position-specific tremor or isometric tremor. Details of the prevention, alleviation or/and treatment of tremor by Lacosamide are described in WO 2006/000397, the disclosure of which is incorporated herein by reference.

It is preferred that tremor does not include Parkinsonian tremor syndromes.

Psychosis includes, but is not limited to, schizophrenia, psychosis associated with bipolar disorder, autism, Alzheimer's disease, attention deficit hyperactivity disorder, drug or/and alcohol abuse, affective disorders, dyskinesias and related disorders, dementia, mental retardation, polydipsia/hyponatraemia, severe personality disorder, acute episodes of mania, obsessive compulsive disorder, intractable chronic insomnia, Huntington's Disease, Tourette's syndrome, Parkinson's disease or/and dopaminergic therapy of Parkinson's disease.

Details of the prevention, alleviation or/and treatment of schizophrenia in an add-on therapy by Lacosamide are described in PCT/EP2006/000722, the disclosure of which is incorporated herein by reference.

Preferably, the CRMP-associated disease of the present invention is not psychosis, in particular not schizophrenia.

Arthritis or an arthritic condition includes, but is not limited to osteoarthritis such as inflammatory osteoarthritis and non-inflammatory osteoarthritis, rheumatoid arthritis including juvenile rheumatoid arthritis, fibromyalgia and a musculoskeletal disorder of one or more joints of a subject including arthritis associated with or secondary to conditions not necessary primarily arthritic. Osteoarthritis can be idiopathic or primary in origin, or secondary to other conditions. Arthritic conditions and conditions to which arthritis can be secondary as used herein are defined herein in the context of pain associated with arthritis or an arthritic condition.

In the context of the present invention, "CRMP" includes any CRMP polypeptide, in particular any CRMP polypeptide described in the state of the art, such as CRMP-1, CRMP-2, CRMP-3, CRMP-4 and CRMP-5 including splice variants thereof. For instance, two splice variants of CRMP-1 have been described.

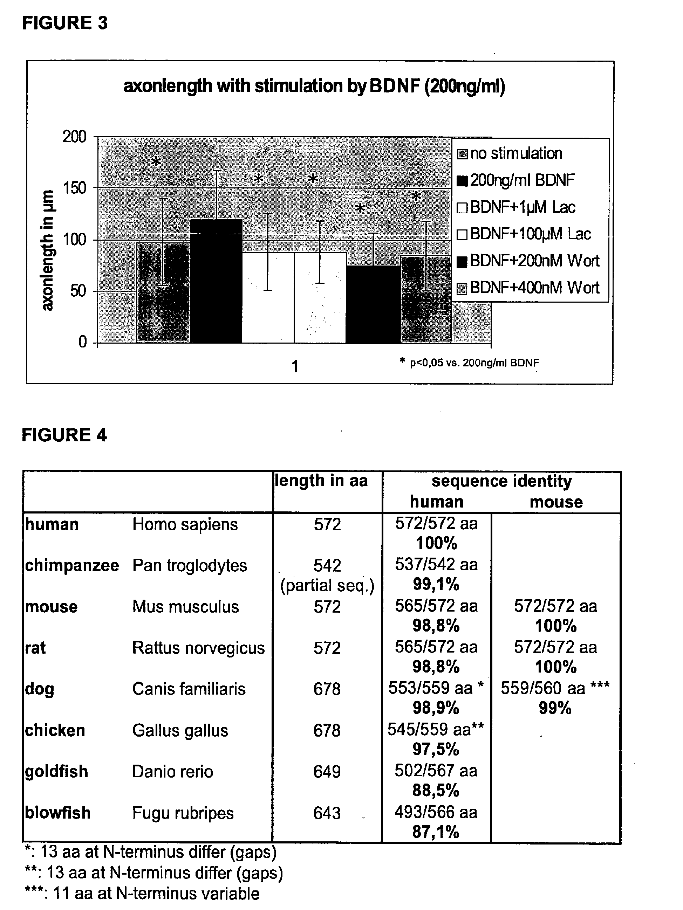

Figure 4 demonstrates that, for instance, CRMP-2 is highly conserved among vertebrate species, in particular among mammalian species. Preferably, CRMP is obtained from a vertebrate species, more preferably from a mammalian species. "CRMP" also includes any nucleic acid encoding a CRMP polypeptide.

"CRMP" also includes a modified CRMP polypeptide. As used herein, "modified CRMP polypeptide" includes but is not limited to modifications of the CRMP sequence as defined herein. Further, "modified CRMP polypeptide" includes but is not limited to post-translational and chemical modifications of CRMP. The person skilled in the art knows suitable methods of chemically modifying a polypeptide, for instance by conjugation at functional groups of amino acid side chains.

SEQ ID NO: 1 describes a nucleotide sequence encoding a CRMP-1 (Genbank NM_001014809.1, GI:62422570). SEQ ID NO: 1 describes a first splice variant of a CRMP-1.

SEQ ID NO: 2 describes a CRMP-1 encoded by SEQ ID NO: 1.

SEQ ID NO: 3 describes a nucleotide sequence encoding another CRMP-1 (Genbank NM_001313.3, GI:62422567). SEQ ID NO: 3 describes a second splice variant of a CRMP-1.

The CRMP-1 nucleic acid preferably comprises SEQ ID NO:1 or/and 3 or a fragment thereof.

SEQ ID NO: 4 describes a CRMP-1 encoded by SEQ ID NO: 3.

The CRMP-1 polypeptide preferably comprises SEQ ID NO:2 or/and 4 or a fragment thereof.

SEQ ID NO: 5 describes a nucleotide sequence encoding a CRMP-2 (Genbank BC056408.1, GI:33991633). The CRMP-2 nucleic acid preferably comprises SEQ ID NO:5 or a fragment thereof.

SEQ ID NO: 6 describes a CRMP-2 encoded by SEQ ID NO: 5. The CRMP-2 polypeptide preferably comprises SEQ ID NO:6 or a fragment thereof.

SEQ ID NO: 7 describes a nucleotide sequence encoding a CRMP-3 (Genbank NM_006426.1, GI:11321616). The CRMP-3 nucleic acid preferably comprises SEQ ID NO:7 or a fragment thereof.

SEQ ID NO: 8 describes a CRMP-3 encoded by SEQ ID NO: 7. The CRMP-3 polypeptide preferably comprises SEQ ID NO:8 or a fragment thereof.

SEQ ID NO: 9 describes a nucleotide sequence encoding a CRMP-4 (Genbank NM_001387.2, GI:45505175). The CRMP-4 nucleic acid preferably comprises SEQ ID NO:9 or a fragment thereof.

SEQ ID NO: 10 describes a CRMP-4 encoded by SEQ ID NO: 9. The CRMP-4 polypeptide preferably comprises SEQ ID NO:10 or a fragment thereof.

SEQ ID NO: 11 describes a nucleotide sequence encoding a CRMP-5 (Genbank NM_020134.2, GI:19923820). The CRMP-5 nucleic acid preferably comprises SEQ ID NO:11 or a fragment thereof.

SEQ ID NO: 12 describes a CRMP-5 encoded by SEQ ID NO: 11. The CRMP-5 polypeptide preferably comprises SEQ ID NO:12 or a fragment thereof.

"CRMP" also includes complementary sequences to the sequences described herein. For instance, transcripts to be translated are complementary to the respective coding DNA sequences. Further, complementary sequences may serve as template for synthesis of a nucleic acid to be transcribed. Thus, complementary sequences of SEQ ID Nos: 1, 3, 5, 7, 9, and 11 and fragments thereof are also suitable in the method of the present invention. The complementary sequences to SEQ ID Nos: 1, 3, 5, 7, 9, and 11 may encode for a polypeptide of SEQ ID Nos: 2, 4, 6, 8, 10, and 12, respectively.

In the method of the present invention, it is preferred that CRMP comprises
(i) a polypeptide selected from the group consisting of SEQ ID NO:2, SEQ ID NO:4, SEQ ID NO:6, SEQ ID NO:8, SEQ ID NO:10, SEQ ID NO:12 and fragments thereof, or/and
(ii) a polypeptide which is at least 70%, preferably at least 80%, more preferably at least 90%, even more preferably at least 95%, most preferably at least 98% identical to the polypeptide of (i).

In the method of the present invention, it is also preferred that CRMP comprises
(iii) a polypeptide encoded by a nucleotide sequence selected from the group consisting of SEQ ID NO:1, SEQ ID NO:3, SEQ ID NO:5, SEQ ID NO:7, SEQ ID NO:9, SEQ ID NO:11, and fragments thereof,
(iv) a polypeptide encoded by a sequence complementary to the nucleotide sequence of (iii),
(v) a polypeptide encoded by a nucleic acid which is at least 70%, preferably at least 80%, more preferably at least 90%, even more preferably at least 95%, most preferably at least 98% identical to the nucleic acid of (iii) or/and (iv),
(vi) a polypeptide encoded by a nucleic acid corresponding to the nucleic acid of (iii), (iv) or/and
(v) within the scope of the degeneracy of the genetic code, or/and
(vii) a polypeptide encoded by a nucleic acid capable of hybridizing with the nucleic acid of (iii), (iv), (v) or/and (vi) under stringent conditions.

It is preferred that the polypeptide of (iv) encodes a polypeptide of (iii).

A preferred CRMP is CRMP-2.

It is preferred that the polypeptide of (i) is selected from SEQ ID NO: 6 and fragments thereof.

It is also preferred that the polypeptide of (iii) is encoded by a nucleotide sequence selected from SEQ ID NO:5 and fragments thereof.

Identity refers to the proportion of identical positions of two sequences (either nucleotide sequences or amino acid sequences) in the region of maximal overlap. A person skilled in the art is aware of methods for identity determination. Identity may be determined by commonly know algorithms such as FASTA or/and BLAST (in particular blastp or blastn for comparison of nucleotide or amino acid sequences; blastx for comparison of a translated nucleotide sequence with an amino acid sequence or tblastn for comparison of an amino acid sequence with a translated nucleotide sequence).

A person skilled in the art is familiar with stringent hybridization conditions (see e. g. Sambrook J. et al., 1989, Molecular Cloning, A Laboratory Manual, Cold Spring Harbor Press, Plainview, N.Y). Preferably, hybridization under stringent conditions means that, after washing for 1 h with 1 x SSC and 0.1% SDS at 50°C, preferably at 55°C, more preferably at 62°C and most preferably at 68° C, particularly for 1 h in 0.2 x SSC and 0.1 % SDS at 50°C, preferably at 55°C, more preferably at 62°C, and most preferably at 68°C, a positive hybridization signal is observed.

The nucleic acid fragment of the present invention preferably has a length of at least 30 nucleotides, more preferably at least 150 nucleotides, even more preferably at least 300 nucleotides, most preferably at least 600 nucleotides.

The nucleic acid fragment of the present invention preferably has a length of the natural CRMP truncated by at least 1 nucleotide, more preferably has a length of at the maximum 1500 nucleotides, even more preferably at the maximum 1200 nucleotides, most preferably at the maximum 900 nucleotides.

The polypeptide fragment of the present invention preferably has a length of at least 10 amino acids, more preferably at least 50 amino acids, even more preferably at least 100 amino acids, most preferably at least 200 amino acids.

The polypeptide fragment of the present invention preferably has a length of the natural CRMP truncated by at least 1 amino acid, more preferably has a length of at the maximum 500 amino acids, even more preferably at the maximum 400 amino acids, most preferably at the maximum 300 amino acids.

Modulation of CRMP in the context of the present invention includes the binding of a substance to CRMP or/and modulation of the biological activity of CRMP. The modulator of CRMP of the present invention may be an antagonist or may be an agonist of the biological activity of CRMP. Preferably, the modulator of CRMP is an antagonist. The biological activity may be modulated by modulation of nucleic acid activity such as transcription or/and translation, or may be modulated by modulating the activity of a translated protein. The modulator may increase or decrease the biological activity of CRMP directly or indirectly by modulation of members of the signalling cascade regulating CRMP activity, in particular of the CRMP-2 signalling cascade, such as GSK-3beta, Akt, PDK, P13-Kinase.

The biological activity of CRMP, in particular CRMP-2, to be modulated includes, but is not limited to, spatial and temporal axogenesis and dendrite formation, formation of multiple axons by overexpression of CRMP, preferably of CRMP-2, in contrast to axons normally having a single axon and several dendrites, in particular in hippocampal neurons, suppression of axonal growth in particular in hippocampal neurons by inhibition of CRMP, preferably of CRMP-2, neuronal apoptosis, interaction with PLD₂, in particular by amino acid position 243-300 (with reference to rat CRMP-2), inhibition of PLD₂ activity, phosphorylation of CRMP, preferably of CRMP-2, dephosphorylation of CRMP, preferably of CRMP-2, activation by dephosphorylation of CRMP, preferably of CRMP-2, inactivation by phosphorylation of CRMP, preferably of CRMP-2, regulation of microtubule assembly in axons by phosphorylation/dephosphorylation, in particular by regulation of the polymerization rate by the tubulin dimer pool or/and stabilization of the microtubule polymer.

In step (a) of the present invention, the substance of the present invention is selected. The method of the present invention does not require limitations upon the substance to be selected. Any substance may be selected. Preferably, the substance is selected from compounds which have a low toxicity for mammalian cells. The substance may be an organic molecule, in particular a small organic compound with a molecular weight of at least about 50 Daltons, preferably at least about 100 Daltons, or/and at the maximum about 2,500 Daltons, preferably at the maximum about 1000 Daltons. The substance may comprise at least one functional group capable of structurally interacting with CRMP, particularly forming hydrogen bonding(s), which group may be selected from the amine, carbonyl, hydroxyl and carboxyl group. The substance may be a biomolecule, which can be selected from peptides, polypeptides, proteins, saccharides, fatty acids, steroids, purines, pyrimidines, nucleic acids including aptamers and siRNA molecules, and derivatives, structural analogs or combinations thereof.

In step (b) of the method of the present invention, the substance selected in step (a) is contacted with CRMP.

In a preferred embodiment of the method of the present invention, in step (b) the substance is contacted with isolated CRMP. In this embodiment, the method of the present invention is a molecular screening assay.

In the context of the present invention, isolated CRMP refers to CRMP partially or completely purified from a cell or/and a tissue expressing CRMP, or a cell or/and tissue lysate comprising CRMP, or a composition comprising CRMP. The cell or/and the tissue may be a natural cell or/and tissue capable of expressing CRMP, or may be a cell or/and tissue modified by recombinant DNA technology capable of expressing CRMP, in particular capable of overexpression CRMP. The cell or/and tissue may be of eukaryotic origin, or the cell may be a prokaryotic cell, preferably a gram-negative cell, such as *E*. *coli.* A person skilled in the art familiar with the amino acid sequence of CRMP or the nucleotide sequence encoding CRMP as described above is able to provide isolated CRMP by isolation from natural sources or by culturing a cell or/and tissue under conditions suitable for the expression and recovery of CRMP.

In step (b), CRMP or/and a member of the CRMP signalling cascade may be bound to a solid phase and contacted with the substance. In this event, at least the substance may be labelled, so that the label can directly or indirectly provide a detectable signal when the substance forms a complex immobilized on the solid phase via CRMP or/and a member of the CRMP signalling cascade. Alternatively, the substance may be bound to a solid phase. In this case, at least CRMP or/and a member of the CRMP signalling cascade may be labelled, so that the complex immobilized via the substance can be detected by the label. Binding on the solid phase may be irreversible or reversible by tagging CRMP, a member of the CRMP signalling cascade or/and the substance and capturing by an solid-phase bound affinity ligand specific for the tag. Suitable tag/ligand pairs are familiar to a person skilled in the art, e.g. biotin-avidin/streptavidin.

In yet another preferred embodiment of the method of the present invention, the substance is contacted in step (b) with a cell capable of expressing or overexpressing CRMP or/and with a non-human animal comprising a cell capable of expressing or overexpressing CRMP. In this embodiment, the method of the present invention is a cellular screening assay.

Step (c) of the method of the present invention refers to determination of CRMP interaction with the substance, wherein a substance interacting with CRMP is a modulator of CRMP.

The interaction of CRMP with the substance may be a direct interaction or an indirect interaction.

A direct interaction includes complex formation. In one embodiment of the present invention, the substance may form a complex with CRMP based upon of binding, in particular specific binding, of the substance with CRMP. In a molecular screening assay, the substance may form a complex with the isolated CRMP. In a cellular screening assay, the substance may form a complex with the CRMP within the cell or/and the non-human animal.

A CRMP complex within the cell or/and the non-human animal or a complex of isolated CRMP may be detected by an antibody against CRMP or CRMP linked antigens. The antibody may be a polyclonal antibody or a monoclonal antibody, recombinant antibody, e.g. a single chain antibody, or a fragment thereof which contain at least one antigen-binding site, e.g. a proteolytic antibody fragment such as a Fab, Fab' or F(ab')₂ fragment or a recombinant antibody fragment such as a scFv fragment. The antibody may also be a chimeric antibody, a humanized antibody or a human antibody.

It is therefore preferred that in step (c), the determination of interaction of isolated CRMP with the substance is the determination of a complex of CRMP with the substance. In particular, the interaction of the isolated CRMP with the substance may be determined by affinity chromatography, e.g. by a pull-down assay.

In a pull-down assay, the physical interaction between two compounds can be determined. In the context of the present invention, the minimal requirement for a pull-down assay is the provision of isolated and tagged CRMP (the bait), which will be immobilized on a solid phase by the tag and used to capture and 'pull-down' the substance to be tested as a protein-binding partner (the prey) by release of the complex formed by bait and prey.

Classical binding assays, classical competition assays, affinity chromatography, etc, which may form part of the method of the present invention, in particular of steps (b) and (c), are familiar to a person skilled in the art.

Complex formation may also take place in a cellular screening assay. It is therefore also preferred that in step (c), the determination of interaction of CRMP with the substance within the cell or/and the non-human animal is the determination of a complex of CRMP with the substance.

In another embodiment of the present invention, the interaction of CRMP with the substance in step (c) is an indirect interaction. Indirect interactions of CRMP with the substance may be employed in a molecular screening assay or a cellular screening assay.

The indirect interaction may be mediated by GSK3beta or other members of the signalling cascade involved in the regulation of CRMP activity, in particular CRMP-2 activity. These members include but are not limited to trk receptor A, trk receptor B, trk receptor C, phosphoinositide 3 (P13) kinase, PDK kinase, Akt kinase, glycogen synthase kinase-3β (GSK-3β). Activation of at least one of trk receptor A, trk receptor B, trk receptor C, P13 kinase, PDK kinase, and Akt kinase result in inactivation of GSK-3β, which leads to an activation of CRMP-2 or other CRMP polypeptides. Inactivation of GSK-3β also leads to an activation of CRMP-2 or other CRMP polypeptides. Inactivation of at least one trk receptor A, trk receptor B, trk receptor C, P13 kinase, PDK kinase, and Akt kinase result in activation of GSK-3β, which leads to an inactivation of CRMP-2 or other CRMP polypeptides. Activation of GSK-3β also leads to an inactivation of CRMP-2 or other CRMP polypeptides.

The interaction of CRMP with the substance may be determined in step (c) by modulation of the biological activity of CRMP within the cell or/and the non-human animal, in particular where, in step (b), a cell capable of expressing or overexpressing CRMP or/and a non-human animal comprising a cell capable of expressing or overexpressing CRMP is used for contacting the substance with CRMP.

Modulation of the biological activity CRMP may be caused by direct interaction. For instance, the modulation may be caused by a complex of CRMP and a substance as described herein. Modulation of the biological activity of CRMP may also be caused by an indirect interaction as described herein, e.g. an indirect interaction of the substance with at least one member of the signalling cascade involved in the regulation of CRMP activity, in particular CRMP-2 activity, as described herein.

Preferred biological activities of CRMP, in particular of CRMP-2, which may be modulated in the method of the present invention are described above. It is more preferred that the biological activity is neuronal polarity, i.e. a neuronal cell is capable of developing at least one axon and dendrite(s). CRMP modulation, in particular CRMP-2 modulation, may be determined by modulation of axon growth in such cells, wherein multiple axons are formed, for example, and the number of dendrites is reduced when CRMP activity, in particular CRMP-2 activity, increases, and short or no axons are formed when CRMP activity, in particular CRMP-2 activity, decreases. Suitable neuronal cell displaying neuronal polarity are hippocampal neurons, in particular cultured hippocampal neurons.

In the method of the present invention, the biological activity of CRMP may be modulated on the nucleic acid level, e.g. by modulating transcription or/and translation of CRMP or/and a member of the CRMP signalling cascade. Modulation of transcription includes a partial or complete gene inactivation, e.g. by gene disruption. Modulation also includes gene activation. Modulation of translation includes application of anti-sense molecules, ribozymes, or/and siRNA molecules. Suitable anti-sense molecules may be selected from DNA molecules, RNA molecules or nucleic acid analogues. Ribozymes may be selected from RNA molecules and nucleic acid analogues. Small RNA molecules capable of RNA interference (siRNA molecules) may be selected from RNA molecules and nucleic acid analogues. The person skilled in the art knows methods for construction of suitable anti-sense molecules or/and siRNA molecules. A suitable sequence may be selected from sequences encoding CRMP such as SEQ ID NO:1, SEQ ID NO:3, SEQ ID NO:5, SEQ ID NO:7, SEQ ID NO:9, SEQ ID NO: 11, and sequences encoding CRMP signalling members, such as trk receptor A, trk receptor B, trk receptor C, P13 kinase, PDK kinase, Akt kinase, GSK-3β, and fragments thereof.

In the method of the present invention, at least the substance may be labelled, so that the label can directly or indirectly provide a detectable signal. Alternatively, at least CRMP or/and at least one member of the CRMP signalling cascade may be labelled. Labelling of the substance, CRMP or/and at least one member of the CRMP signalling cascade can be performed by a moiety providing a detectable signal, for instance a luminescence signal, in particular a fluorescence or phosphorescence signal. Suitable dyes which can serve as luminescence labels are familiar to a person skilled in the art.

In yet another preferred embodiment, the method of the present invention is performed in a high throughput format. In this embodiment, the substances to be tested for interaction with CRMP are provided in libraries of synthetic or/and natural compounds. A person skilled in the art is aware of the technology of cellular and molecular screening assays in the high throughput format.

Yet another subject of the present invention is a cell capable of overexpressing or underexpressing CRMP or/and expressing a modified CRMP polypeptide. The cell of the present invention may be any cell capable of expressing CRMP when cultured in a suitable medium under such conditions that expression of CRMP takes place.

In a preferred embodiment, the cell is a eukaryotic cell, such as a fungal cell (e.g. yeast), an insect cell (such as Sf9), a non-human animal cell (such as CHO, COS, Hek293 or BHK), a human cell (such as HeLa), or a plant cell. More preferably, the eukaryotic cell is a neuronal cell, e.g. a hippocampal or a dorsal root ganglion cell, which may display neuronal polarity.

In another preferred embodiment, the cell is a prokaryotic cell which recombinantly expresses CRMP, more preferably a gram-negative prokaryotic cell, most preferably E. coli.

Preferably, the cell of the present invention may be a transgenic cell, i.e. a cell carrying a heterologous CRMP gene or fragment thereof.

The cell of the present invention may be used for the provision of isolated CRMP (see above), or may be used in step (b) or/and (c) of the method of the present invention for contacting the substance selected in step (a) with CRMP.

Yet another subject of the present invention is a non-human animal comprising a cell capable of overexpressing or underexpressing CRMP or/and expressing a modified CRMP polypeptide. Preferably, the non-human animal of the present invention may be a transgenic animal, i.e. an animal carrying a heterologous CRMP gene or fragment thereof or a CRMP gene with modified nucleotide sequence i.e. point mutations modifying protein activity.

CRMP, in particular CRMP-2 is naturally found in neuronal tissues, in particular during development of the central nervous system, but can also be found in peripheral tissues such as adult testis, lung tissue of fetal mouse, or monocytes (Charrier et al., Mol. Neurobiology 2003; 28(1): 51-63). The non-human animal of the present invention may underexpress or overexpress CRMP in these cells or/and tissues. Further, the non-human animal of the present invention may express CRMP in cells or/and tissues where it is not normally expressed, such as in tissues different from neuronal tissue, adult testis, lung tissue of fetal mouse and monocytes. The non-human animal of the present invention may also express CRMP in cells or/and tissues at times where it is normally not expressed, or may underexpress CRMP in cells or/and tissues at times where it is normally expressed. Since expression of CRMP is low in adult neuronal tissue, overexpression of CRMP may be induced in adult neuronal tissue. On the other hand, CRMP may be underexpressed in developing neuronal tissue.

Non-human animals of the present invention may be obtained by homologous recombination in embryonic stem (ES) cells, where the normal locus of CRMP is mutated. Alternatively, a nucleic acid construct encoding CRMP may be injected into oocytes for randomly integrating into the genome. An ES cell line may be employed, or ES cells may be obtained freshly from a host, e.g. mouse, rat, guinea pig, etc. Construction of animals carrying recombinant DNA by ES cell manipulation is a technology familiar to a person skilled in the art.

Non-human animals of the present invention may be obtained by random mutagenesis of the animal genome, induced by the treatment with mutagenic compounds such as the chemical N-ethyl-N-nitrosourea (ENU) and a subsequent breading of animals carrying homozygous mutations in the CRMP gene locus. Random chemical mutagenesis and breeding of specific mutant aninmals is a technology familiar to a person skilled in the art.

If the manipulation of CRMP leads to lethality of the embryo during development, cells, tissues or/and organs of the embryo may be maintained as grafts or transplants, or by in vitro culture.

The non-human animals of the present invention may be any non-human mammal, such as a laboratory animal, domestic animals, etc, for example, mouse, rat, guinea pig, sheep, cow, pig and others.

The non-human animal of the present invention may be used for the provision of isolated CRMP (see above), or may be used in step (b) or/and (c) of the method of the present invention for contacting the substance selected in step (a) with CRMP.

As demonstrated herein, Lacosamide modulates neurotrophin-induced neuronal outgrowth. Thus, Lacosamide can thereby attenuate the induction or/and progression of neurological diseases such as epilepsy or/and neuropathic pain. Thus a compound of the present invention as described herein suitable for the prevention, alleviation or/and treatment of a CRMP associated disease, in particular Lacosamide, can have disease modifying properties when interacting with CRMP, in particular CRMP-2. This may explain the potential long-term neuroprotective effects of lacosamide.

Thus a further aspect of the present invention is a method for identification of compounds which are disease-modifying compounds or/and compound suitable for neuroprotective treatment, in particular long-term neuroprotective treatment, comprising
(a) selecting a substance,
(b) contacting the substance selected in step (a) with CRMP, and
(c) determining whether CRMP interacts with the substance,
wherein a substance interacting with CRMP is a disease-modifying compound or/and a compound suitable for neuroprotective treatment, in particular long-term neuroprotective treatment. It is preferred that the disease-modifying compound or/and the compound suitable for neuroprotective treatment is suitable for treatment of a CRMP associated disease. As used in this context, "CRMP associated disease" includes the preferred CRMP associated diseases as described herein. In this context, a particularly preferred CRMP associated diseases is a neurological disease, such as epilepsy or a pain syndrome, e. g. neuropathic pain.

In the context of the present invention, "disease-modifying" refers to the ability of a compound to attenuate the induction or/and progression of a disease, in particular a CRMP associated disease as defined herein.

Another aspect of the present invention is the use of a compound of Formula (I), (II) or/and (III) as described herein for the manufacture of a disease-modifying medicament or/and a medicament suitable for neuroprotective treatment such as long-term neuroprotective treatment, in particular in a CRMP associated disease as described herein. As used in this context, "compound of Formula (I), (II) or/and (III)" includes the preferred compounds as described herein. A most preferred compound is lacosamide. As used in this context, "CRMP associated disease" includes the preferred CRMP associated diseases as described herein. In this context, a particularly preferred CRMP associated diseases is a neurological disease, such as epilepsy or a pain syndrome, e. g. neuropathic pain.

Yet another subject of the present invention are modulators of CRMP suitable for the prevention, alleviation or/and treatment of a CRMP-associated disease which are compounds of Formula (I) 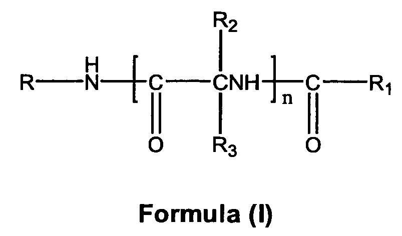 wherein
R is hydrogen, alkyl, alkenyl, alkynyl, aryl, aryl alkyl, heterocyclic, heterocyclic alkyl, alkyl heterocyclic, cycloalkyl or cycloalkyl alkyl, and R is unsubstituted or is substituted with at least one electron withdrawing group, or/and at least one electron donating group;
R₁ is hydrogen or alkyl, alkenyl, alkynyl, aryl alkyl, aryl, heterocyclic alkyl, alkyl heterocyclic, heterocyclic, cycloalkyl, cycloalkyl alkyl, each unsubstituted or substituted with at least one electron donating group or/and at least one electron withdrawing group;
   and
R₂ and R₃ are independently hydrogen, alkyl, alkenyl, alkynyl, alkoxy, alkoxyalkyl, aryl alkyl, aryl, halo, heterocyclic, heterocyclic alkyl, alkyl heterocyclic, cycloalkyl, cycloalkyl alkyl, or Z-Y wherein R₂ and R₃ may be unsubstituted or substituted with at least one electron withdrawing group or/and at least one electron donating group;
Z is O, S, S(O)ₐ, NR₄, NR'₆, PR₄ or a chemical bond;
Y is hydrogen, alkyl, aryl, aryl alkyl, alkenyl, alkynyl, halo, heterocyclic, heterocyclic alkyl, alkyl heterocyclic and Y may be unsubstituted or substituted with at least one electron donating group or/and at least one electron withdrawing group, provided that when Y is halo, Z is a chemical bond, or
ZY taken together is NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇or N⁺R₅R₆R₇, 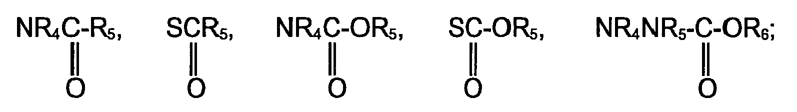
R'₆ is hydrogen, alkyl, alkenyl, or alkenyl which may be unsubstituted or substituted with at least one electron withdrawing group or/and at least one electron donating group;
R₄, R₅ and R₆ are independently hydrogen, alkyl, aryl, aryl alkyl, alkenyl, or alkynyl, wherein R₄, R₅ and R₆ may independently be unsubstituted or substituted with at least one electron withdrawing group or/and at least one electron donating group;
R₇ is R₆ or COOR₈ or COR₈, which R₇ may be unsubstituted or substituted with at least one electron withdrawing group or/and at least one electron donating group;
R₈ is hydrogen or alkyl, or aryl alkyl, and the aryl or alkyl group may be unsubstituted or substituted with at least one electron withdrawing group or/and at least one electron donating group; and
n is 1-4; and
a is 1-3.

In a preferred embodiment, the compound of Formula (I) has the general Formula (II), 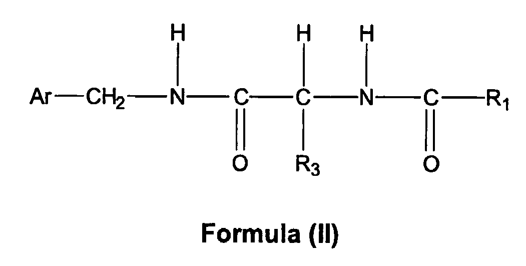 wherein
- Ar: is aryl which is unsubstituted or substituted with at least one electron donating group or/and at least one electron withdrawing group, preferably halo, more preferably fluoro;
- R₁: is alkyl, preferably alkyl containing 1-3 carbon atoms, more preferably methyl; and
- R₃: is as defined herein.

In a more preferred embodiment, the compound of Formulae (I) or/and (II) has the general Formula (III), 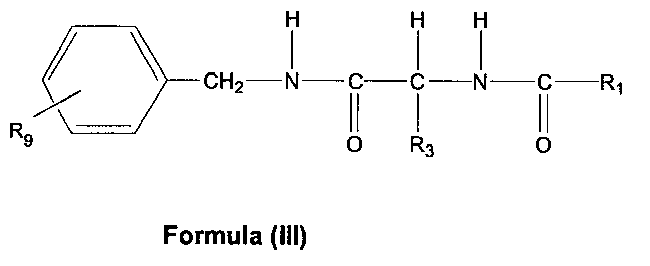 wherein
- R₉: is one or more substituents independently selected from the group consisting of hydrogen, halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide;
- R₃: is selected from the group consisting of hydrogen, alkyl, arylalkyl, alkoxy, alkoxyalkyl, aryl, heterocyclic, heterocyclic alkyl, N-alkoxy-N-alkylamino, N-alkoxyamino, and N-carbalkoxy; and
- R₁: is alkyl, preferably alkyl containing 1 to 3 carbon atoms, more preferably methyl.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

As used herein, the term configuration particularly refers to the configuration around the carbon atom to which R₂ and R₃ or H and R₃ are attached, even though other chiral centers may be present in the molecule. Therefore, when referring to a particular configuration, such as D or L, it is to be understood to mean the D or L stereoisomer at the carbon atom to which R₂ and R₃ or H and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which R₂ and R₃ or H and R₃ are attached). These stereoisomers may be found in mixtures of the L and D stereoisomer, e.g., racemic mixtures. The D stereoisomer is preferred.

It is preferred that the compounds of Formula (I) are in the R configuration. It is also preferred that the compounds of Formula (II) are in the R configuration. It is also preferred that the compounds of Formula (III) are in the R configuration.

It is preferred that the compounds of Formulae (I), (II) or/and (III) in the R configuration are substantially enantiopure. As used herein, the term "substantially enantiopure" refers to a content of the R enantiomer of at least 99.5%. This corresponds to an enantiomeric excess (ee) of 99%. The respective quantities of R and S enantiomer may be determined by chiral column chromatography, e.g. by HPLC with "ChiralPak" as chiral, stationary phase.

The term "alkyl" (alone or in combination with another term(s)) means a straight- or branched-chain saturated hydrocarbyl substituent preferably containing from 1 to about 20 carbon atoms (C₁-C₂₀-alkyl), more preferably from 1 to about 8 carbon atoms (C₁-C₈-alkyl), even more preferably from 1 to about 6 carbon atoms (C₁-C₆-alkyl), and most preferably from 1 to 3 carbon atoms (C₁-C₃-alkyl). The alkyl groups include methyl, ethyl, propyl, isopropyl, butyl, isobutyl, tertiary butyl, amyl, hexyl, and the like. Further, alkyl groups also include halogenated alkyl groups up to perhalogenation, e.g. trifluoromethyl, if not indicated otherwise.

The term "alkoxy" (alone or in combination with another term(s)) refers to -O-alkyl and means a straight- or branched-chain alkoxy substituent preferably containing from 1 to about 20 carbon atoms (C₁-C₂₀-alkoxy), more preferably from 1 to about 8 carbon atoms (C₁-C₈-alkoxy), even more preferably from 1 to about 6 carbon atoms (C₁-C₆-alkoxy), and most preferably from 1 to 3 carbon atoms (C₁-C₃-alkoxy). The alkoxy groups include methoxy, ethoxy, propoxy, butoxy, isobutoxy, tert-butoxy, pentoxy, hexoxy and the like. Further, alkoxy groups include halogenated alkoxy groups up to perhalogenation, if not indicated otherwise.

The term "alkoxyalkyl" refers to an alkyl group substituted with at least one alkoxy group. The alkoxyalkyl groups include methoxymethyl (-CH₂-OCH₃) groups, methoxyethyl (-CH₂-CH₂-OCH₃) groups, ethoxymethyl (-CH₂-O-CH₂CH₃) groups and the like.

The term "N-alkoxyamino" refers to amino groups substituted with one or two alkoxy groups, e.g. -NH-N(OCH₃)₂.

The term "N-alkoxy-N-alkylamino" refers to amino groups substituted with an alkoxy group and an alkyl group, e.g. -N(CH₃)(OCH₃), -N(CH₃)(OCH₂-CH₃) and the like.

The term "N-carbalkoxy" refers to amino groups substituted with a carbalkoxy group, e.g. -NH(C(O)-O-CH₃), -NH(C(O)O-CH₂-CH₃).

The term "aryl", when used alone or in combination with other term(s), refers to an aromatic group which contains from 6 up to 18 ring carbon atoms (C₆-C₁₈-aryl), preferably from 6 up to 10 ring carbon atoms (C₆-C₁₀-aryl), and includes polynuclear aromatics. The aryl groups may be monocyclic, bicyclic, tricyclic or polycyclic and may be fused rings. A polynuclear aromatic compound as used herein, is meant to encompass bicyclic and tricyclic fused aromatic ring systems containing from 10-18 ring carbon atoms. Aryl groups include phenyl and polynuclear aromatics e.g., naphthyl, anthracenyl, phenanthrenyl, azulenyl and the like. The aryl group also includes groups such as ferrocenyl. Aryl groups may be unsubstituted or mono or polysubstituted with electron withdrawing or/and electron donating groups. A preferred aryl group is phenyl, which may unsubstituted or mono or polysubstituted with electron withdrawing or/and electron donating groups.

The term "aryl alkyl" as used herein alone or in combination with other term (s) means an alkyl group as defined herein carrying an aryl substitutent as defined herein. Preferred aryl alkyl groups are aryl-C₁-C₆-alkyl, aryl-C₁-C₃-alkyl, C₆-C₁₀-aryl-alkyl, C₆-C₁₀-aryl-C₁-C₆-alkyl, C₆-C₁₀-aryl-C₁-C₃-alkyl. More preferred aryl alkyl groups are phenyl-C₁-C₆-alkyl and phenyl-C₁-C₃-alkyl. Even more preferred aryl alkyl groups include, for example, benzyl, phenylethyl, phenylpropyl, phenylisopropyl, phenylbutyl, diphenylmethyl, 1,1-diphenylethyl, 1,2-diphenylethyl, and the like. Most preferred is benzyl.

The term "alkenyl" (alone or in combination with another term(s)) means a straight- or branched-chain alkenyl substituent containing at least one double bond and preferably containing from 2 to about 20 carbon atoms (C₂-C₂₀-alkenyl), more preferably from 2 to about 8 carbon atoms (C₂-C₈-alkenyl), and even more preferably from 2 to about 6 carbon atoms (C₂-C₆-alkenyl), most preferably 2 or 3 carbon atoms (C₂-C₃-alkenyl). The alkenyl group may be in the Z or E form. Alkenyl groups include vinyl, propenyl, 1-butenyl, isobutenyl, 2-butenyl, 1-pentenyl, (Z)-2-pentenyl, (E)-2-pentenyl, (Z)-4-methyl-2-pentenyl, (E)-4-methyl-2-pentenyl, pentadienyl, e.g., 1, 3 or 2,4-pentadienyl, and the like.

The term "alkynyl" (alone or in combination with another term(s)) means a straight- or branched-chain alkynyl substituent containing at least one triple bond and preferably containing from 2 to about 20 carbon atoms (C₂-C₂₀-alkynyl), more preferably from 2 to about 8 carbon atoms (C₂-C₈-alkynyl), and even more preferably from 2 to about 6 carbon atoms (C₂-C₆-alkynyl), most preferably 2 or 3 carbon atoms (C₂-C₃-alkynyl). The alkynyl group includes ethynyl, propynyl, 1-butynyl, 2-butynyl, 1-pentynyl, 2-pentynyl, 3-methyl-1-pentynyl, 3-pentynyl, 1-hexynyl, 2-hexynyl, 3-hexynyl and the like.

The term "cycloalkyl" when used alone or in combination with another term (s) means a cycloalkyl group containing from 3 to 18 ring carbon atoms (C₃-C₁₈-cycloalkyl), preferably from 6 up to 10 ring carbon atoms (C₃-C₁₀-cycloalkyl). The cycloalkyl groups may be monocyclic, bicyclic, tricyclic, or polycyclic, and the rings may be fused. The cycloalkyl may be completely saturated or partially saturated. Examples of cycloalkyl groups include cyclopropyl, cyclobutyl, cyclopentyl, cyclohexyl, cycloheptyl, cyclooctyl, cyclodecyl, cyclohexenyl, cyclopentenyl, cyclooctenyl, cycloheptenyl, decalinyl, hydroindanyl, indanyl, fenchyl, pinenyl, adamantyl, and the like. The cycloalkyl group includes the cis or trans forms. Cycloalkyl groups may be unsubstituted or mono or polysubstituted with electron withdrawing or/and electron donating groups. In a bridged bicyclic cycloalkyl group, the substituents may either be in endo or exo positions.

The term "cycloalkyl alkyl" as used herein alone or in combination with other term(s) means an alkyl group as defined herein carrying a cycloalkyl substitutent as defined herein. Preferred cycloalkyl alkyl groups are cycloalkyl-C₁-C₆-alkyl, cycloalkyl-C₁-C₃-alkyl, C₆-C₁₀-cycloalkyl-alkyl, C₆-C₁₀-cycloalkyl-C₁-C₆-alkyl, C₆-C₁₀-cycloalkyl-C₁-C₃-alkyl. A more preferred cycloalkyl alkyl group is selected from cyclohexyl-C₁-C₆-alkyl and cyclohexyl-C₁-C₃-alkyl.

The term "halo" or "halogen" includes fluoro, chloro, bromo, and iodo.

The prefix "halo" indicates that the substituent to which the prefix is attached is substituted with one or more independently selected halogen radicals. For example, haloalkyl means an alkyl substituent wherein at least one hydrogen radical is replaced with a halogen radical. Examples of haloalkyls include chloromethyl, 1-bromoethyl, fluoromethyl, difluoromethyl, trifluoromethyl, 1,1,1-trifluoroethyl, and the like. Illustrating further, "haloalkoxy" means an alkoxy substituent wherein at least one hydrogen radical is replaced by a halogen radical. Examples of haloalkoxy substituents include chloromethoxy, 1-bromoethoxy, fluoromethoxy, difluoromethoxy, trifluoromethoxy (also known as "perfluoromethyloxy"), 1,1,1,-trifluoroethoxy, and the like. It should be recognized that if a substituent is substituted by more than one halogen radical, those halogen radicals may be identical or different (unless otherwise stated).

The terms "electron-withdrawing" and "electron donating" refer to the ability of a substituent to withdraw or donate electrons, respectively, relative to that of hydrogen if the hydrogen atom occupied the same position in the molecule. These terms are well understood by one skilled in the art and are discussed in Advanced Organic Chemistry, by J. March, John Wiley and Sons, New York, NY, pp.16-18 (1985) and the discussion therein is incorporated herein by reference. Electron withdrawing groups include halo, including bromo, fluoro, chloro, iodo; nitro, carboxy, alkenyl, alkynyl, formyl, carboxyamido, aryl, quaternary ammonium, haloalkyl such as trifluoromethyl, aryl alkanoyl, carbalkoxy and the like. Electron donating groups include such groups as hydroxy, alkoxy, including methoxy, ethoxy and the like; alkyl, such as methyl, ethyl, and the like; amino, alkylamino, dialkyl amino, aryloxy such as phenoxy, mercapto, alkylthio, alkylmercapto, disulfide (alkyldithio) and the like. One of ordinary skill in the art will appreciate that some of the aforesaid substituents may be considered to be electron donating or electron withdrawing under different chemical conditions. Moreover, the present invention contemplates any combination of substituents selected from the above-identified groups.

The electron donating or/and electron withdrawing groups may independently be present in any one of the substituents in Formula (I), (II) or/and (III) e.g., in R, R_{1'} R₂, R₃, R₄, R₅, R₆, R'₆, R₇, R₈, R₉ or/and R₁₀ as defined herein.

The at least one electron withdrawing or/and at least one electron donating group is preferably selected independently from halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, disulfide, alkanoyl, amino alkyl, aryloyl, cyano, sulfonyl, sulfoxide, heterocyclic, guanidine, sulfonium salts, mercaptoalkyl, and alkyldithio.

The term "sulfide" encompasses mercapto, mercapto alkyl and alkylthio, while the term disulfide encompasses alkyldithio.

In the compounds of the present invention, the at least one electron withdrawing or/and at least one electron donating group is more preferably selected independently from halo, alkyl, alkenyl, alkynyl, nitro, carboxy, formyl, carboxyamido, aryl, quaternary ammonium, haloalkyl, aryl alkanoyl, hydroxy, alkoxy, carbalkoxy, amino, alkylamino, dialkylamino, aryloxy, mercapto, alkylthio, alkylmercapto, and disulfide.

Even more preferably, the at least one electron withdrawing or/and at least one electron donating group is selected from halo, C₁-C₆-alkyl, C₂-C₆-alkenyl, C₁-C₆-alkynyl, nitro, carboxy, formyl, carboxyamido, C₆-C₁₀-aryl, quaternary ammonium, C₁-C₆-haloalkyl, C₆-C₁₀-aryl C₂-C₆-alkanoyl, hydroxy, C₁-C₆-alkoxy, C₂-C₆-carbalkoxy, amino, C₁-C₆-alkylamino, C₁-C₆-dialkylamino, C₆-C₁₀-aryloxy, mercapto, C₁-C₆-alkylthio, C₁-C₆-alkylmercapto, and disulfide.

Even more preferably, the electron withdrawing or/and electron donating groups may also be independently selected from halo, C₁-C₆-alkoxy, nitro, carboxy, formyl, carboxyamido, quaternary ammonium, hydroxy, amino, mercapto, and disulfide.

Most preferred electron withdrawing or/and electron donating groups are independently selected from halo such as fluoro and C₁-C₆-alkoxy such as methoxy and ethoxy.

The term "carbalkoxy" as used herein alone or in combination with other term(s) means an -CO-O-alkyl, wherein alkyl is as defined herein, taking into account that the -CO-O- group provides one carbon atom in addition to those of the alkyl group. The carbalkoxy group preferably contains from 2 to about 20 carbon atoms (C₂-C₂₀-carbalkoxy), more preferably from 2 to about 8 carbon atoms (C₂-C₈-carbalkoxy), even more preferably from 2 to about 6 carbon atoms (C₂-C₆-carbalkoxy), and most preferably from 2 to 3 carbon atoms (C₂-C₃-carbalkoxy).

The term "alkanoyl" as used herein alone or in combination with other term (s) means an alkanoyl group -CO-alkyl, wherein alkyl is as defined herein, taking into account that the -CO- group provides one carbon atom in addition to those of the alkyl group. The alkanoyl preferably contains from 2 to about 20 carbon atoms (C₂-C₂₀-alkanoyl), more preferably from 2 to about 8 carbon atoms (C₂-C₈-alkanoyl), even more preferably from 2 to about 6 carbon atoms (C₂-C₆-alkanoyl), and most preferably from 2 to 3 carbon atoms (C₂-C₃-alkanoyl). The alkanoyl group may be straight chained or branched. The alkanoyl groups include, for example, formyl, acetyl, propionyl, butyryl, isobutyryl, tertiary butyryl, pentanoyl and hexanoyl.

As employed herein, a heterocyclic group contains at least one heteroatom in the cyclic structure, preferably one, two, three or four heteroatoms. The at least one heteroatom may be independently selected from sulfur, nitrogen and oxygen. The heterocyclic groups contemplated by the present invention include heteroaromatics and saturated and partially saturated heterocyclic groups. The heterocyclics may be monocyclic, bicyclic, tricyclic or polycyclic and may be fused rings. The heterocyclics also include the so-called benzoheterocyclics. Heterocyclic groups may be unsubstituted or mono or polysubstituted with electron withdrawing or/and electron donating groups. The heterocyclic groups preferably contain up to 18 ring atoms and up to a total of 17 ring carbon atoms and may be unsubstituted or mono or polysubstituted with electron withdrawing or/and electron donating groups.

More preferably, the heterocyclic group may be independently selected from 5 or 6-membered monocyclic heterocyclic groups and may be unsubstituted or mono or polysubstituted with electron withdrawing or/and electron donating groups. The heterocyclic group may also be more preferably selected independently from furyl, thienyl, pyrazolyl, pyrrolyl, methylpyrrolyl, imidazolyl, indolyl, thiazolyl, oxazolyl, isothiazolyl, isoxazolyl, piperidyl, pyrrolinyl, piperazinyl, quinolyl, triazolyl, tetrazolyl, isoquinolyl, benzofuryl, benzothienyl, morpholinyl, benzoxazolyl, tetrahydrofuryl, pyranyl, indazolyl, purinyl, indolinyl, pyrazolindinyl, imidazolinyl, imadazolindinyl, pyrrolidinyl, furazanyl, N-methylindolyl, methylfuryl, pyridazinyl, pyrimidinyl, pyrazinyl, pyridyl, epoxy, aziridino, oxetanyl, azetidinyl, the N-oxides of the nitrogen containing heterocycles, such as the N-oxides of pyridyl, pyrazinyl, and pyrimidinyl and the like. Even more preferably, the heterocyclic moieties are those aforementioned heterocyclics which are monocyclic.

The heterocyclics may also be more preferably selected independently from thienyl, furyl, pyrrolyl, benzofuryl, benzothienyl, indolyl, oxazolyl, methylpyrrolyl, morpholinyl, pyridiyl, pyrazinyl, imidazolyl, pyrimidinyl, and pyridazinyl. Especially preferred heterocyclic are independently selected from furyl, oxazolyl, pyridyl, pyrazinyl, imidazolyl, pyrimidinyl, and pyridazinyl. The most preferred heterocyclics are independently selected from furyl, pyridyl and oxazolyl.

The monocyclic 5- or 6-membered heterocyclic groups in the compounds of the present invention are preferably of the Formula (IV): 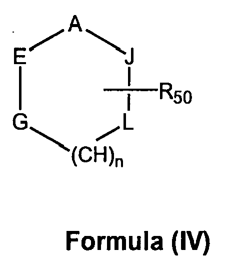 or those corresponding partially or fully saturated form thereof, wherein n is 0 or 1; and
R₅₀ is H, an electron withdrawing group or an electron donating group;
A, E, L, J and G are independently CH, or a heteroatom selected from the group consisting of N, O, S; but when n is 0, G is CH, or a heteroatom selected from the group consisting of NH, O and S with the proviso that at most two of A, E, L, J and G are heteroatoms.

When n is 0, the above heteroaromatic moiety is a five membered ring, while if n is 1, the heterocyclic moiety is a six membered monocyclic heterocyclic moiety.

If the ring depicted in Formula (IV) contains a nitrogen ring atom, then the N-oxide forms are also contemplated to be within the scope of the invention.

When R₂ or R₃ is a heterocyclic of Formula (IV), it may be bonded to the main chain by a ring carbon atom. When n is 0, R₂ or R₃ may additionally be bonded to the main chain by a nitrogen ring atom.

The term "heterocyclic alkyl" as used herein alone or in combination with other term(s) means an alkyl group as defined above carrying a heterocyclic substituent as defined above. Preferred heterocyclic alkyl groups are heterocyclic-C₁-C₆-alkyl, heterocyclic-C₁-C₃-alkyl, wherein the heterocyclic may be a preferred, more preferred or most preferred heterocyclic group as defined herein.

The term "alkyl heterocyclic" as used herein alone or in combination with other term(s) means a heterocyclic group as defined above carrying at least one alkyl substituent as defined above. Preferred alkyl heterocyclic groups are C₁-C₆-alkyl-heterocyclic, C₁-C₃-alkyl-heterocyclic, wherein the heterocyclic group may be a preferred, more preferred or most preferred heterocyclic group as defined herein.

The preferred compounds are those wherein n is 1, but di (n=2), tri (n=3) and tetrapeptides (n=4) are also contemplated to be within the scope of the invention.

In the ZY groups representative of R₂ or/and R₃, in the formula (I) or/and (II), Z may be O, S, S(O)ₐ, wherein a is 1-3, NR₄, NR'₆, PR₄ or a chemical bond; and Y may be hydrogen, alkyl, aryl, aryl alkyl, alkenyl, alkynyl, halo, heterocyclic, heterocyclic alkyl, alkyl heterocyclic, and Y may be unsubstituted or substituted with at least one electron donating group or/and at least one electron withdrawing group, provided that when Y is halo, Z is a chemical bond, or
ZY taken together may be NR₄NR₅R₇, NR₄OR₅, ONR₄R₇, OPR₄R₅, PR₄OR₅, SNR₄R₇, NR₄SR₇, SPR₄R₅, PR₄SR₇, NR₄PR₅R₆, PR₄NR₅R₇ or N⁺R₅R₆R₇, 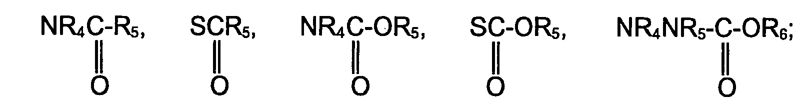 wherein R₄, R₅, R'₆, R₆, R₇, are as defined herein.

The ZY groups representative of R₂ or/and R₃ in the Formula (I) or/and (II) may be hydroxy, alkoxy, such as methoxy, ethoxy, aryloxy, such as phenoxy; thioalkoxy, such as thiomethoxy, thioethoxy; thioaryloxy such as thiophenoxy; amino; alkylamino, such as methylamino, ethylamino; arylamino, such as anilino; dialkylamino, such as, dimethylamino; trialkyl ammonium salt, hydrazino; alkylhydrazino and arylhydrazino, such as N-methylhydrazino, N-phenylhydrazino, carbalkoxy hydrazino, aralkoxycarbonyl hydrazino, aryloxycarbonyl hydrazino, hydroxylamino, such as N-hydroxylamino (-NH-OH), alkoxy amino [(NHOR₁₈) wherein R₁₈ is alkyl], N-alkylhydroxyl amino [(NR₁₈)OH wherein R₁₈ is alkyl], N-alkyl-O-alkylhydroxyamino, i.e., [N(R₁₈)OR₁₉ wherein R₁₈ and R₁₉ are independently alkyl], and O-hydroxylamino (-O-NH₂); alkylamido such as acetamido; trifluoroacetamido; alkoxyamino, (e.g., NH(OCH₃); and heterocyclicamino, such as pyrazoylamino.

In a preferred ZY group, Z is O, NR₄ or PR₄; Y is hydrogen or alkyl.

In another preferred embodiment,
ZY is NR₄R₅R₇, NR₄OR₅, ONR₄R₇,

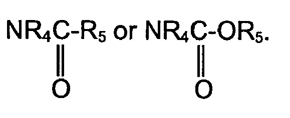

In a more preferred that ZY is NR₄OR₅, or ONR₄R₇.

Another more preferred ZY is N-hydroxyamino, N-alkylhydroxyamino, N-alkyl-O-alkyl hydroxyamino, O-alkylhydroxyamino, N-alkoxy-N-alkylamino, N-alkoxyamino, or N-carbalkoxy.

In Formula (I), R is preferably aryl or aryl alkyl, more preferably R is aryl alkyl, wherein R is unsubstituted or substituted with at least one electron donating group or/and at least one electron withdrawing group. R may be phenyl or benzyl, most preferably benzyl, wherein R is unsubstituted or substituted with at least one electron donating group or/and at least one electron withdrawing group. If R is substituted, R is preferably substituted on the aryl ring. In this embodiment, the at least one electron donating group or/and at least one electron withdrawing group is preferably halo, more preferably fluoro.

In Formulae (I), (II) or/and (III), R₁ is H or alkyl. More preferably, R₁ is alkyl, preferably containing from 1 to 6 carbon atoms, more preferably containing from 1 to 3 carbon atoms. Most preferably the R₁ group is methyl. R₁ may be unsubstituted or substituted with at least one electron donating group or/and at least one electron withdrawing group.

Further, it is preferred that one of R₂ and R₃ is hydrogen. It is more preferred that R₂ is hydrogen. Other preferred moieties of R₂ in Formula (I) are aryl such as phenyl, aryl alkyl such as benzyl, and alkyl. It is to be understood that the preferred groups of R₂ may be unsubstituted or mono or poly substituted with electron donating or/and electron withdrawing groups. It is preferred that the at least one electron withdrawing or/and at least one donating group in R₂ is independently alkoxy, N-hydroxyamino, N-alkylhydroxyamino, N-alkyl-O-alkyl hydroxyamino or O-alkylhydroxyamino, and especially methoxy or ethoxy.

In Formulae (I), (II) or/and (III), R₃ may be hydrogen, an alkyl group unsubstituted or substituted by at least an electron donating or/and at least one electron withdrawing group, an aryl group unsubstituted or substituted by at least an electron donating or/and at least one electron withdrawing group heterocyclic, heterocyclic alkyl, or ZY.

It is preferred that R₃ is hydrogen, alkyl unsubstituted or substituted by at least an electron donating or/and at least one electron withdrawing group, aryl which is unsubstituted or substituted by at least one electron donating group or/and at least one electron withdrawing group, heterocyclic, heterocyclic alkyl or ZY, wherein Z is O, NR₄ or PR₄; Y is hydrogen or alkyl; ZY is NR₄NR₅R₇, NR₄OR₅, ONR₄R₇,

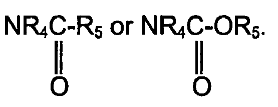

It is also preferred that R₃ is alkyl unsubstituted or substituted by at least an electron donating or/and at least one electron withdrawing group; or Z-Y, wherein Z-Y is as defined herein.

It is also preferred that R₃ is alkyl unsubstituted or substituted by at least an electron donating or/and at least one electron withdrawing group; NR₄OR₅, or ONR₄R₇, wherein R₄, R₅ and R₇ are as defined herein.

It is also preferred that R₃ is CH₂-Q, wherein Q is alkoxy especially containing 1-3 carbon atoms; or R₃ is NR₄OR₅ or ONR₄R₇, wherein R₄, R₅, and R₇ are as defined herein.

R₃ is also preferably alkyl which is unsubstituted or substituted with at least one alkoxy especially containing 1-3 carbon atoms.

R₃ is also preferably CH₂-Q, wherein Q is alkoxy preferably containing 1-3 carbon atoms, more preferably Q is ethoxy or methoxy.

R₃ is also preferably NR₄OR₅, or ONR₄R₇, wherein R₄, R₅ and R₇ are as defined herein, and R₄, R₅ and R₇ are as defined herein, e.g. N-alkoxy, N-alkoxy-N-alkylamino or N-carbalkoxy.

R₃ is also preferably heterocyclic, heterocyclic alkyl, or aryl, which may be unsubstituted or substituted with at least an electron donating or/and at least one electron withdrawing group. A most preferred heterocyclic in R₃ is furyl or oxazolyl.

R₃ is also preferably selected from the group consisting of hydrogen, alkyl, arylalkyl such as benzyl, alkoxy, alkoxyalkyl, aryl such as phenyl, heterocyclic, heterocyclic alkyl, N-alkoxy-N-alkylamino, N-alkoxyamino and N-carbalkoxy.

It is to be understood that the preferred groups of R₃ may be unsubstituted or mono or poly substituted with electron donating or/and electron withdrawing groups. It is preferred that the at least one electron withdrawing or/and at least one electron donating group in R₃ is independently alkoxy, N-hydroxyamino, N-alkylhydroxyamino, N-alkyl-O-alkyl hydroxyamino or O-alkylhydroxyamino, and especially methoxy or ethoxy.

R₄, R₅, R₆, R'₆, R₇ and R₈ are preferably independently hydrogen or alkyl.

R₄, R₅, and R₇ are preferably independently hydrogen or alkyl preferably containing 1-3 carbon atoms.

The most preferred aryl is phenyl. The most preferred halo is fluoro.

In the compounds of Formula (I), R is preferably aryl alkyl, wherein R is unsubstituted or substituted with at least one electron donating group or/and at least one electron withdrawing group.

In the compounds of Formula (I), R₁ is preferably alkyl which is unsubstituted or substituted with at least one electron donating group or/and at least one electron withdrawing group.

In the compounds of Formula (I), R₂ and R₃ is preferably independently hydrogen, alkyl which is unsubstituted or substituted by at least one electron donating group or/and at least one electron withdrawing group, aryl which is unsubstituted or substituted by at least one electron donating group or/and at least one electron withdrawing group, heterocyclic, heterocyclic aryl, or ZY; wherein Z is O, NR₄ or PR₄; and Y is hydrogen or
alkyl; or ZY is NR₄NR₅R₇, NR₄OR₅, ONR₄R₇, 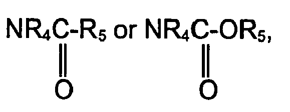 wherein R₄, R₅ and R₇ are as defined herein.

In the compounds of Formula (I), the preferred groups of R₂ and R₃ may be unsubstituted or mono or poly substituted with electron donating or/and electron withdrawing groups, such as alkoxy (e.g., methoxy, ethoxy, and the like), N-hydroxyamino, N-alkylhydroxyamino, N-alkyl-O-alkyl hydroxyamino and O-alkylhydroxyamino.

In the compounds of Formula (I), the at least one electron donating group or/and at least one electron withdrawing group in R₂ or/and R₃ is preferably independently hydroxy or alkoxy.

It is more preferred that in the compounds of Formula (I), R₂ is hydrogen.

In the compounds of Formula (II), R₁ is preferably methyl.

In preferred compounds of Formula (II), R₃ is hydrogen or alkyl unsubstituted or substituted by at least one electron donating group or/and at least one electron withdrawing group; or R₃ is heterocyclic, heterocyclic alkyl, or Z-Y, wherein Z-Y and heterocyclic are as defined herein.

In other preferred compounds of Formula (II), R₃ is an alkyl group which is unsubstituted or substituted by at least one electron donating group or/and at least one electron withdrawing group, NR₄OR₅ or ONR₄R₇, wherein R₄, R₅ and R₇ are as defined herein and wherein the at least one electron donating group or/and at least one electron withdrawing group is preferably selected from hydroxy and alkoxy.

In further preferred compounds of Formula (II), R₃ is CH₂-Q, wherein Q is alkoxy preferably containing 1-3 carbon atoms, more preferably methoxy, or R₃ is NR₄OR₅ or ONR₄R₇ wherein R₄, R₅ and R₇ are independently hydrogen or alkyl containing 1-3 carbon atoms.

In other preferred compounds of Formula (II), R₃ is -CH₂-Q, wherein Q is alkoxy containing 1 to 3 carbon atoms.

In the compounds of Formula (II), Ar is preferably phenyl unsubstituted or substituted with at least one halo, preferably with at least one fluoro. More preferably Ar in Formula (II) is unsubstituted phenyl.

In preferred compounds of Formula (III), R₉ is hydrogen or fluoro, R₃ is selected from the group consisting of methoxymethyl, phenyl, N-methoxy-N-methylamino, and N-methoxyamino, and R₁ is methyl.

The most preferred compounds of the present invention include:
(R)-2-acetamido-N-benzyl-3-methoxy-propionamide;
(R)-2-acetamido-N-benzyl-3-ethoxy-propionamide;
O-methyl-N-acetyl-D-serine-m-fluorobenzyl-amide;
O-methyl-N-acetyl-D-serine-p-fluorobenzyl-amide;
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide;
D-α-acetamido-N-(2-fluorobenzyl)-2-furanacetamide;
D-α-acetamido-N-(3-fluorobenzyl)-2-furanacetamide.

It is to be understood that the various combinations and permutations of the Markush groups of R₁, R₂, R₃, R and n described herein are contemplated to be within the scope of the present invention. Moreover, the present invention also encompasses compounds and compositions which contain one or more elements of each of the Markush groupings in R₁, R₂, R₃, n and R and the various combinations thereof. Thus, for example, the present invention contemplates that R₁ may be one or more of the substituents listed hereinabove in combination with any and all of the substituents of R₂, R₃, and R with respect to each value of n.

More preferred is a compound of Formula (I), (II) or/and (III) in the R configuration, preferably substantially enantiopure, wherein the substituent R is benzyl which is unsubstituted with at least one halo group, wherein R₃ is CH₂-Q, wherein Q is alkoxy containing 1-3 carbon atoms and wherein R₁ is methyl. Preferably R is unsubstituted benzyl or benzyl substituted with at least one halo group which is a fluoro group.

Depending upon the substituents, the present compounds may form addition salts as well. All of these forms are contemplated to be within the scope of this invention including mixtures of the stereoisomeric forms.

The manufacture of compounds utilized in the present invention is described in U.S. Patent Nos. 5,378,729 and 5,773,475, and in the international application PCT/EP 2005/010603 the contents of which are incorporated by reference.

The compounds utilized in the present invention are useful as such as depicted in the Formulae (I), (II) or/and (III) or can be employed in the form of salts in view of its basic nature by the presence of the free amino group. Thus, the compounds of Formulae (I), (II) or/and (III) form salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric, metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

The invention is further illustrated by the following figures and examples. The examples present evidence that Lacosamide binds directly to CRMP-2.

### Figure Legend

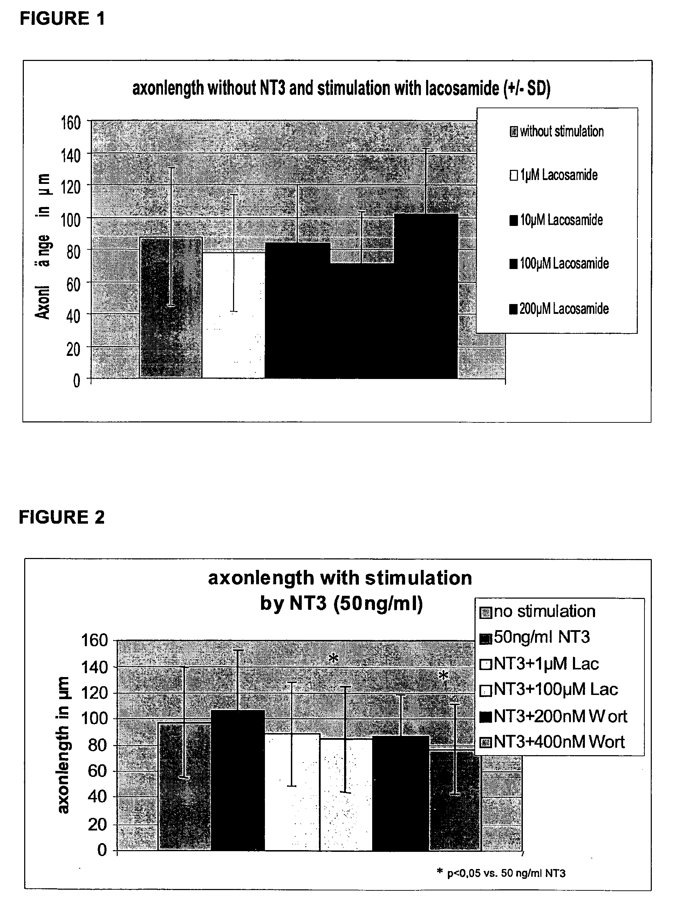

Figure 1 describes the axon length after treatment by lacosamide. Stimulation of hippocampal primary cells by Lacosamide (1-200 µM) does not significantly change basal axonal outgrowth.
Figure 2 describes the axon length after stimulation with 50 ng/ml NT3 and treatment with Lacosamide (Lac) or the kinase inhibitor Wortmannin (Wort). Treatment of neurons with 50 ng/ml NT3 increases axonal outgrowth. Treatment with 100 µM Lacosamide (Lac) or 400 nM of the positive control Wortmannin (Wort) significantly decreases NT3-induced axonal growth.
Figure 3 describes Axon length after stimulation with 200 ng/ml BDNF and treatment with Lacosamide (Lac) or the kinase inhibitor Wortmannin (Wort). Treatment of neurons with 200 ng/ml BDNF significantly increases axonal outgrowth. Treatment with 1 µM and 100 µM Lacosamide (Lac) or 200 nM and 400 nM of the positive control Wortmannin (Wort) significantly decreases BDNF-induced axonal growth.
Figure 4 gives an overview upon CRMP-2 amino acid sequence identity among various vertebrate species (at least 87 % identical to the human CRMP-2 sequence). In the mammalian species considered, the CRMP-2 sequences are at least 98 % identical to the human CRMP-2 sequence.

### Example 1

### Lacosamide, a new analgesic and anti-epileptic drug, interacts with the collapsin response mediator protein, CRMP-2.

The aim of the experiment was to identify proteins which interact with Lacosamide to resolve the molecular mechanism of action of Lacosamide in disease treatment.

Biological pull-down experiments were performed by incubating a set of four Lacosamide-related affinity-reagents with biotinyl-moieties (R)-N-Benzyl-2-acetamido-6-[Nε-(biotinyl)-2-isothiocynato-6-aminohexanamido]-aminohexanamide((R)-7), (S)-N-Benzyl-2-acetamido-6-[Nε-(biotinyl)-2-isothiocynato-6-aminohexanamido]-aminohexanamide((S)-7), (R)-N-{3-[Nε-(biotinyl)-2-isothiocynato-6-aminohexanamide]-benzyl}-2-acetamido-3-methoxypropion-amide((R)-34) and (R)-N-{4-[Nε-(biotinyl)-2-isothiocynato-6-aminohexanamide]-benzyl}-2-acetamido-3-methoxypropion-amide((R)-35) to rat brain. Isolation of fractions led to enrichment of an overlapping set of proteins, including collapsing response mediator protein (CRMP-2) and some additional proteins related to the vesicle release machinery.

Several findings suggest that CRMP, in particular CRMP-2 is indeed a target of Lacosamide:
(a) The pattern of affinity captured proteins is completely different from raw extracts and controls and the protein is consistently captured under a variety of conditions.
(b) There appears to be some competition of affinity tags with the Lacosamide.
(c) Differences between active R- and less active S-enantiomers of Lacosamide can be found in 2D gels, their exact molecular nature however would require an in-depth mass spectrometry approach.

### Example 2

### CRMP-2 expression in Xenopus oocytes: Binding study with Lacosamide

The aim of this study was to confirm the interaction of Lacosamide with CRMP-2 in a classical competition protein binding experiment.

Based on the results of the previous study, the present binding curve ranged from 1 to 100 µM (10 concentrations, in triplicates). With crude membranes from CRMP-2 transfected Xenopus oocytes, competitive and specific binding was observed. The KD-value appeared to be below 5 µM; Bₘₐₓ was about 200 pM/mg. Here the low specific radio activity of ¹⁴C-labelled Lacosamide potentially sets a limit to further binding experiments.

### Example 3

### Influence of Lacosamide on axon growth in hippocampal primary cells

It is known that CRMP-2 is critical for specifying axon/dendrite fate, possibly by promoting neurite elongation via microtubule assembly. Neurotrophins e.g. NT-3 and BDNF decrease phosphorylated CRMP-2, thereby promoting axonal outgrowth. Phosphorylation of CRMP-2 through GSK-3β and thus inactivation of CRMP-2 was shown to inhibit neurotropin-induced axonal outgrowth.

To get further insight in the implications of the potential functional interaction of Lacosamide and CRMP-2, the effects of Lacosamide on the neurotrophin-induced neuronal differentiation were analyzed in vitro. It could be shown that the neurotrophins NT3 and BDNF stimulate axonal outgrowth in a primary culture of embryonal hippocampal neurons. Quantification of neurite outgrowth was performed by immunofluorescence microscopy employing an antibody against the axonal marker protein Tau-1. BDNF and NT3 induced but not basal neuronal outgrowth was attenuated by Lacosamide (Figure 2 and 3). Lacosamide alone did not significantly change basal axonal outgrowth (Figure 1). The findings indicate a functional interaction of Lacosamide and CRMP-2 antagonizing CRMP-2 activity.

## Claims

1. A method for identifying a modulator of CRMP suitable for the prevention, alleviation or/and treatment of a CRMP associated disease, comprising the steps
(a) selecting a substance,
(b) contacting the substance selected in step (a) with CRMP, and
(c) determining whether CRMP interacts with the substance,
wherein a substance interacting with CRMP is a modulator of CRMP.

2. The method of claim 1, wherein CRMP comprises
(i) a polypeptide selected from the group consisting of SEQ ID NO:2, SEQ ID NO:4, SEQ ID NO:6, SEQ ID NO:8, SEQ ID NO:10, SEQ ID NO:12 and fragments thereof, or/and
(ii) a polypeptide which is at least 70% identical to the polypeptide of (i).

3. The method of claim 2, wherein the polypeptide of (i) is selected from SEQ ID NO: 6 and fragments thereof.

4. The method of claim 1 or 2, wherein CRMP comprises
(iii) a polypeptide encoded by a nucleotide sequence selected from the group consisting of SEQ ID NO:1, SEQ ID NO:3, SEQ ID NO:5, SEQ ID NO:7, SEQ ID NO:9, SEQ ID NO:11, and fragments thereof,
(iv) a polypeptide encoded by a sequence complementary to the nucleotide sequence of (iii),
(v) a polypeptide encoded by a nucleic acid which is at least 70% identical to the polypeptide of (iii) or/and (iv),
(vi) a polypeptide encoded by a nucleic acid corresponding to the nucleic acid of (iii), (iv) or/and (v) within the scope of the degeneracy of the genetic code, or/and
(vii) a polypeptide encoded by a nucleic acid capable of hybridizing with the nucleic acid of (iii), (iv), (v) or/and (vi) under stringent conditions.

5. The method of claim 4, wherein the polypeptide of (iii) is encoded by a nucleotide sequence selected from SEQ ID NO:5 and fragments thereof.

6. The method of any of the preceding claims, wherein CRMP is CRMP-2.

7. The method of any of the preceding claims, wherein the CRMP associated disease is selected from neurological diseases, psychiatric diseases, or/and inflammatory diseases.

8. The method of any of the preceding claims, wherein the CRMP associated disease is selected from epilepsy, pain syndromes, motoneuron disorders, dyskinesias, tremor syndromes, psychosis, especially schizophrenia, and arthritis or an arthritic condition.

9. The method of claim 8, wherein the CRMP associated disease is selected from epilepsy, pain syndromes, motoneuron disorders, dyskinesias, tremor syndromes different from parkinsonian tremor syndrome, and arthritis or an arthritic condition.

10. The method of claim 8 or 9, wherein the CRMP associated disease is selected from epilepsy, pain syndromes, and arthritis or an arthritic condition.

11. The method of claim 10, wherein the CRMP associated disease is *status epilepticus.*

12. The method of claim 10, wherein the CRMP associated disease is painful diabetic neuropathy, preferably associated with Diabetes mellitus Type I or II, more preferably Type II.

13. The method of claim 10, wherein the CRMP associated disease is pain associated with arthritis or with an arthritic condition.

14. The method of any of the preceding claims, wherein the CRMP associated disease is a CRMP-2 associated disease.

15. The method of any of the claims 1 to 14, wherein the modulator is an agonist.

16. The method of any of the claims 1 to 14, wherein the modulator is an antagonist.

17. The method of any of the claims 1 to 16, wherein in step (b), the substance is contacted with isolated CRMP.

18. The method of claim 17, wherein in step (c), a complex of CRMP with the substance is determined.

19. The method of claim 17 or 18 wherein in step (c), the interaction of the isolated CRMP with the substance is determined by affinity chromatography.

20. The method of any of the claims 1 to 16, wherein in step (b), the substance is contacted with a cell capable of expressing or overexpressing CRMP or/and with a non-human animal comprising a cell capable of expressing or overexpressing CRMP.

21. The method of claim 20, wherein in step (c), a complex of CRMP with the substance is determined.

22. The method of claims 20 or 21, wherein in step (c) the interaction of CRMP with the substance is an indirect interaction.

23. The method of claim 22, wherein the indirect interaction is mediated by trk receptor A, trk receptor B, trk receptor C, P13 kinase, PDK kinase, Akt kinase, or/and GSK-3β.

24. The method of any of the claims 20 to 23, wherein in step (c), the interaction of CRMP with the substance is determined by modulation of the biological activity of CRMP within the cell or/and the non-human animal.

25. The method of claim 24, wherein the cell is a neuronal cell displaying neuronal polarity.

26. The method of claim 24 or 25, wherein in step (c), the interaction of CRMP with the substance is determined by modulation of axon growth.

27. The method of any of the preceding claims performed in a high throughput format.

28. A cell capable of overexpressing or underexpressing CRMP or/and expressing a modified CRMP polypeptide.

29. The cell of claim 28, which is a eukaryotic cell, preferably a neuronal cell.

30. The cell of claim 28 or 29 which is a transgenic cell.

31. A non-human animal comprising a cell capable of overexpressing or underexpressing CRMP or/and expressing a modified CRMP polypeptide.

32. The animal of claim 31 which is a transgenic animal.

33. A method for identification of compounds which are disease-modifying compounds or/and compound suitable for neuroprotective treatment, in particular long-term neuroprotective treatment, comprising
(a) selecting a substance,
(b) contacting the substance selected in step (a) with CRMP, and
(c) determining whether CRMP interacts with the substance,
wherein a substance interacting with CRMP is a disease-modifying compound or/and a compound suitable for neuroprotective treatment, in particular long-term neuroprotective treatment.

34. The use of a compound of Formula (I), (II) or/and (III) for the manufacture of a disease-modifying medicament or/and a medicament for neuroprotective treatment, in particular long-term neuroprotective treatment.

35. The use of claim 34 wherein the compound is lacosamide.
